# EUROPEAN PATENT SPECIFICATION

(11) **EP 4 034 661 B1**
(45) Date of publication and mention of the grant of the patent: **06.09.2023**
(21) Application number: 21715779.1
(22) Date of filing: 11.03.2021
(51) Int. Cl.: C12N 15/113, A61K 31/7105, C12N 9/22

(54) **ALL-IN-ONE AAV VECTORS FOR TREATING CORONAVIRUS-INDUCED DISEASES**
ALL-IN-ONE AAV-VEKTOREN ZUR BEHANDLUNG VON CORONAVIRUS-INDUZIERTEN ERKRANKUNGEN
VECTEURS D'AAV TOUT-EN-UN POUR LE TRAITEMENT DE MALADIES INDUITES PAR CORONAVIRUS

(30) Priority: 08.04.2020 US 202063006996 P
(43) Date of publication of application: 03.08.2022
(73) Proprietor: Georg-August-Universitaet Goettingen Stiftung Oeffentlichen Rechts, Universitaetsmedizin, 37075 Goettingen (DE)
(72) Inventor: ZEISBERG, Elisabeth, 37085 Goettingen (DE); XU, Xingbo, 37077 Goettingen (DE); ZEISBERG, Michael, 37085 Goettingen (DE); HASENFUSS, Gerd, 37077 Goettingen (DE); TAN, Xiaoying, 37077 Goettingen (DE)
(74) Representative: Grund, Martin
(86) International application number: PCT/EP2021/056221
(87) International publication number: WO 2021/204492

(56) References cited:
- WO-A1-2019/040664
- WO-A1-2021/183887
- TIMOTHY R ABBOTT ET AL: "Development of CRISPR as a prophylactic strategy to combat novel coronavirus and influenza", BIORXIV, 14 March 2020 (2020-03-14), pages 1-82, XP055733410, DOI: 10.1101/2020.03.13.991307 cited in the application
- NGUYEN TUAN M ET AL: "Virus against virus: a potential treatment for 2019-nCov (SARS-CoV-2) and other RNA viruses", CELL RESEARCH, SPRINGER SINGAPORE, SINGAPORE, vol. 30, no. 3, 18 February 2020 (2020-02-18), pages 189-190, XP037049278, ISSN: 1001-0602, DOI: 10.1038/S41422-020-0290-0 [retrieved on 2020-02-18]
- SILVANA KONERMANN ET AL: "Transcriptome Engineering with RNA-Targeting Type VI-D CRISPR Effectors", CELL, vol. 173, no. 3, 19 April 2018 (2018-04-19), pages 665-676, XP055529705, Amsterdam NL ISSN: 0092-8674, DOI: 10.1016/j.cell.2018.02.033 cited in the application

## Description

### TECHNICAL FIELD OF THE INVENTION

The present invention relates to the field of biomedicine. Specifically, the invention provides AAV vectors comprising sequences encoding Cas13d and guide RNAs for cleaving single stranded viral RNA, including the provision of such guide RNAs.

### BACKGROUND OF THE INVENTION

The recent and ongoing outbreak of the novel coronavirus SARS-CoV-2 has resulted in a dramatically high incidence of infections and deaths worldwide. The basic reproduction number (*R*₀) of the virus (approximately 3) will inevitably result in an even higher increase in the number of coronavirus cases. The SARS-CoV-2 viral strain is known to be extremely infectious and primarily spreads through the respiratory tract, by droplets or respiratory secretions. An infection with SARS-CoV-2 often leads to pronounced lung injury and to severe acute respiratory syndrome. SARS-CoV-2 is a member of the coronavirus family (*Coronaviridae*), which is an extensive family of single-stranded RNA viruses, frequently considered to cause a variety of human illness and disease ranging from the common cold to acute diseases such as MERS (MERS-CoV) and SARS (SARS-CoV) [1]. Although there exists an urgent need for an effective clinical treatment strategy, there are currently no available curative or preventative therapies or a promising drug candidate for treating SARS-CoV-2.

CRISPR/Cas systems that can effectively target and cleave single stranded RNAs (ssRNAs) may potentially offer a therapeutic approach against SARS-CoV-2. Considering the various Cas proteins, Cas13, in particular the Cas13d variant, appears to be a promising candidate for such an approach since recent studies have highlighted the ability of Cas13 to efficiently and specifically target and cleave ssRNAs in several model systems, including in mammalian cells [2]. First considerations to target SARS-CoV-2 using Cas13d instead of Cas9 have recently been published [12].

Thus, the present invention provides a new drug candidate and therapeutic regimen for treating infections caused by viruses of the *Coronaviridae* family, in particular, infections arising from SARS-CoV, MERS-CoV, and the recently identified SARS-CoV-2, by initiating Cas13-mediated cleavage of single stranded RNAs to infected mammalian cells. Accordingly, the invention provides for guide RNAs that guide the Cas13 protein, preferably a Cas13d protein, to their respective target sites in the genome of viruses derived from the *Coronaviridae* family, in particular, of the family members SARS-CoV, MERS-CoV and SARS-CoV-2. The invention further provides for AAV vectors comprising Cas13 guide RNAs for introducing Cas13, preferably Cas13d, to human cells.

### SUMMARY OF THE INVENTION

The present invention describes the use of Cas13 targeting and cleavage of single-stranded RNA to target and cleave the genome of a single stranded RNA virus of the *Coronaviridae* family, particularly of the family members MERS-CoV, SARS-CoV and SARS-CoV-2. Guide RNAs associated with Cas13, preferably Cas13d, may have their target site positioned in regions conserved between members of the family of *Coronaviridae,* ORF1ab, S, E, M and N. AAV vectors comprising a Cas13d as well as a guide RNA expression cassette are used as a vehicle for the transport of Cas13d into a cell infected with a virus.

Thus, a guide RNA for use with Cas13 having less than 1000 amino acids, preferably for use with a Cas13d, is provided, wherein the guide RNA target site is a sequence comprised by the SARS-CoV-2 virus.

Tthe guide RNA target site may be a sequence that is conserved between the genomes of human-associated viruses of the *Coronaviridae* family.

The guide RNA target site may be a sequence conserved between the genomes of SARS-CoV-2, MERS-CoV and SARS-CoV.

The guide RNA target site may be a sequence comprised by one or more of the Orflab, S, E, M and N regions in the respective genomes of SARS-CoV-2, MERS-CoV and SARS-CoV.

The guide RNA sequence may comprise a sequence that is selected from the group consisting of SEQ ID NO:1 to SEQ ID NO:39.

Specifically, the invention is directed to a guide RNA for use with a Cas13 protein, wherein the guide RNA target site is a sequence comprised by a SARS-CoV-2 genome, wherein the guide RNA sequence comprises a sequence selected from the group consisting of SEQ ID NOs: 4, 7, 11, 15, 23, 27, 31.

Particularly suitable guide RNAs include the spacer sequences of any of SEQ ID NOs: 4, 7, 15, 23, 27, and 31.

The invention further provides for a nucleic acid molecule comprising a sequence encoding a Cas13d protein and a guide RNA expression cassette encoding a Cas13 guide RNA, wherein the guide RNA sequence is selected from the group consisting of SEQ ID NOs: 4, 7, 11, 15, 23, 27, 31 and comprising a U6 promoter.

In another embodiment, the nucleic acid molecule encodes more than one guide RNA.

In another embodiment, the nucleic acid molecule encodes guide RNAs comprising the sequences of SEQ ID NOs: 4, 7, and 15; SEQ ID NOs: 15, 23, and 31; SEQ ID NOs: 15, 27, and 31; SEQ ID NOs: 23, 27, and 31; SEQ ID NOs: 4, 15, 23, 27, and 31; SEQ ID NOs: 4, 7, 27, and 31; SEQ ID NOs: 4, 23, and 31; SEQ ID NOs: 7, 27, and 31; SEQ ID NOs: 4, 7, 15, 23, 27, and 31.

In another embodiment, the nucleic acid molecule encodes guide RNAs comprising the sequences of SEQ ID NOs: 4, 7, and 15; SEQ ID NOs: 4, 15, 23, 27, and 31; and SEQ ID NOs: 4, 7, 27, and 31.

In another embodiment, the nucleic acid molecule is a plasmid.

In a preferred embodiment, the nucleic acid molecule is a single plasmid.

In another embodiment, the Cas13d protein encoded by said sequence does not comprise a nuclear localization signal (NLS).

In another embodiment, the encoded by said sequence Cas13d protein is a fusion protein comprising an N-terminal binding domain (N-NTD) of the nucleocapsid protein of SARS-CoV-2.

In another embodiment, the nucleic acid molecule is obtainable by inserting a spacer sequence of a guide RNA into plasmid pAAV-U6-gRNA-CMV-Cas13d of SEQ ID NO:40; or by inserting at least one spacer sequence of at least guide RNA into plasmid pAAV-U6-gRNA-CMV-Cas13d-array triguide of SEQ ID NO:41, plasmid pAAV-U6-gRNA-quadguide-CMV-Cas13d-V3-basic of SEQ ID NO:42, plasmid pAAV-U6-gRNA-CMV-Cas13d-Sapl of SEQ ID NO:43, or plasmid pAAV-U6-gRNA-CMV-Cas13d-NTO-Aarl of SEQ ID NO:44.

The invention further provides an AAV vector comprising the nucleic acid molecule.

In a preferred embodiment, the AAV vector is selected from the group of AAV1, AAV2, AAV5, AAV6 and AAV9, preferably an AAV2 vector.

In a further preferred embodiment, the AAV vector is an AAV9 vector.

In another embodiment, the AAV vector backbone has been reduced in size compared to the full-length transcript.

The invention further provides an adenoviral vector comprising the nucleic acid molecule.

The invention also provides for a pharmaceutical composition comprising the AAV vector or the adenoviral vector.

The invention also provides for a pharmaceutical composition comprising at least one guide RNA as described above and at least one mRNA encoding a Cas13 protein.

In one embodiment, the Cas13 protein is a Cas13d protein or a Cas13a protein.

In another embodiment, the Cas13d protein encoded by said mRNA does not comprise a nuclear localization signal (NLS).

In another embodiment, the Cas13d protein encoded by said mRNA is a fusion protein comprising an N-terminal binding domain (N-NTD) of the nucleocapsid protein of SARS-CoV-2.

In a further aspect, the invention also provides an AAV vector, the adenoviral vector, or a pharmaceutical composition as described above for use in treating a human-associated virus caused disease or syndrome.

In one embodiment, the disease or syndrome is the result of an infection with a coronavirus that is genetically related to the group consisting of MERS-CoV, SARS-CoV and SARS-CoV-2.

In a preferred embodiment, the disease is COVID-19.

In another embodiment, upon expression, the Cas13d protein cleaves the human-associated virus.

In a preferred embodiment, the AAV vector, the adenoviral vector, or the pharmaceutical composition is to be administered via the upper respiratory tract, preferably by intranasally or intratracheally administration or in an aerosol composition, for example, by means of an inhaler or nebulizer.

In another preferred embodiment, the AAV vector, the adenoviral vector, or the pharmaceutical composition is to be administered to a patient by means of a ventilator.

In a further preferred embodiment, the AAV vector, the adenoviral vector, or the pharmaceutical composition is to be administered to the myocardium of the patient.

### BRIEF DESCRIPTION OF THE DRAWINGS

**Figure 1** shows a phylogenetic tree schematic of the seven presently known Cas13 proteins, for example, as reported in [3].
**Figure 2** is a schematic illustrating an alignment of genomes derived from SARS-CoV-2, SARS-CoV, and MERS-CoV. The short dashes below the alignment indicate the location of the Cas13d guide RNAs that are designed to target the conserved sequence regions between the genomes SARS-CoV-2, SARS-CoV and MERS-CoV.
**Figure 3** shows a vector map of the AAV2 plasmid comprising the Cas13d encoding sequence as well as a Cas13d guide RNA expression cassette pAAV2-U6-gRNA-CMV-Cas13d. **Figure 3A****:** Vector map featuring a single guide RNA (see SEQ ID NO:40). **Figure 3B****:** Vector map illustrating three insertion sites for the guide RNA spacer sequences (see SEQ ID NO:41).
**Figure 4** illustrates a sequence alignment of the respective genomes derived from SARS-CoV-2, SARS-CoV and MERS-CoV as well as the sequences used for the alignment.
**Figure 5** shows the inhibitory efficiency of single guide RNA constructs of the invention in a luciferase reporter assay.
**Figure 6A** shows a first experimental design for testing guide RNA constructs of the invention in SARS-CoV-2-infected human epithelial lungs cells. **Figure 6B** shows the inhibitory efficiency of several combinations of guide RNA constructs in the experimental design of Figure 6A.
**Figure 7A** shows a second experimental design for testing guide RNA constructs of the invention in SARS-CoV-2-infected human epithelial lungs cells. **Figure 7B** shows the inhibitory efficiency of several combinations of guide RNA constructs in the experimental design of Figure 7A.
**Figure 8** shows the inhibitory efficiency of several combinations of guide RNA constructs and multi-guide RNA constructs in the experimental design of Figure 7A.

### DETAILED DESCRIPTION OF THE INVENTION

The invention is defined by the appended claims.

### Definitions

*AAV vector:* The term "AAV vector" as used herein refers to an adeno-associated virus (AAV) capable of introducing a nucleic acid sequence into target cells. The vector may comprise a sequence encoding Cas13d and/or a guide RNA expression cassette encoding a guide RNA and comprising a U6 promoter.

*Vector backbone:* The term "vector backbone" refers to the native nucleic acid sequences of an AAV vector.

*Cas13d:* As used herein, the term "Cas13d" refers to a Cas endonuclease of a CRISPR/Cas13d system, including the RfxCas13d endonuclease.

*Conserved:* The term "conserved" herein refers to sequences or sequence portions within viral genomes of certain members of the *Coronaviridae* family that are shared by at least two other members of the *Coronaviridae* family.

*Coronavirus:* The term "coronavirus" as used herein refers to any virus of the family of *Coronaviridae.* A coronavirus that is capable of infecting human cells is also referred to herein as a human-associated coronavirus.

*COVID-19:* The term "COVID-19" as used herein refers to the disease known as *Coronavirus Disease 2019,* which is caused by an infection from SARS-CoV-2, a type of coronavirus.

*Derivative:* The term "derivative" as used herein refers to a virus that is closely related to a virus as described herein. In particular, a virus is a derivative of another virus if their respective genomes show a sequence similarity of at least 50%.

*Guide RNA:* The term "guide RNA" is used herein to designate a component of a CRISPR/Cas system. In the present case, "guide RNA" refers to a guide RNA of a CRISPR/Cas13 system, for instance, a Cas13d protein. A guide RNA is a non-coding short RNA sequence that "guides" the Cas protein to its target and cleavage site. The guide RNA comprises a nucleic acid sequence that binds to a complementary target site in a target nucleic acid sequence. By way of example, the target nucleic acid of Cas13d is a single stranded RNA sequence.

*Human-associated virus:* The term "human-associated virus" refers to any virus that is capable of infecting a human cell.

*Infection:* The term "infection" as used herein refers to the invasion of bodily tissues of an organism by pathogenic agents, the multiplication of such agents, as well as the reaction of the host tissue to the pathogenic agents and the toxins they produce. As used herein, the term "infection" refers to a viral infection of a cell.

*Lower respiratory tract:* As used herein, this term refers to the portion of the larynx below the vocal folds, trachea, bronchi, bronchioles and the lungs, including the respiratory bronchioles, alveolar ducts, alveolar sacs, and alveoli.

*MERS:* The term "MERS" refers to the disease *Middle East Respiratory Syndrome,* which is a disease or syndrome caused by an infection from MERS-CoV, a type of coronavirus.

*SARS:* The term "SARS" refers to the disease known as *Severe Acute Respiratory Syndrome,* which is caused by an infection from SARS-CoV, a type of coronavirus.

*Transduction:* The term "transduction" as used herein refers to the deliberate introduction of nucleic acids into a cell by means of a viral vehicle. In particular, "transduction" refers to the introduction of an AAV vector into a cell.

*Upper respiratory tract:* As used herein, this term refers to nose and nasal passages, paranasal sinuses, the pharynx, and the portion of the larynx above the vocal folds.

### Exemplary advantages of the invention

The presently disclosed invention relates to the targeting and cleaving of a viral genome using a Cas13 protein, preferably a Cas13d protein, to thereby inhibit or eliminate the replication capacity of the virus.

The invention therefore provides a guide RNA for guiding a Cas13 protein, preferably a Cas13d protein, to a respective target and cleavage site in the viral genome. The inventors designed the instant guide RNAs based on an observation that coronaviruses transmitted from animals to humans have one or more conserved regions in common, and that these conserved regions may be responsible for the highly infectious capacity attributable to such human-associated coronaviruses. Specifically, the present inventors identified and characterized 31 different guide RNA sequences targeting those highly conserved regions of the respective viral sequences (SEQ ID NO:1 to SEQ ID NO:31).

In the presently known families of Cas proteins, the Class II, Type VI Cas13 is the RNA targeting endonuclease having the smallest size, of approximately 930 amino acids. Cas13d has been shown to possess a high level of catalytic activity and specificity in mammalian cells. Thus, Cas13d appears to be particularly suitable for efficacious transduction and specific targeting and degradation of single stranded RNA viruses, such as coronaviruses.

The invention further provides an AAV vector comprising a nucleic acid molecule comprising sequences encoding for at least one guide RNA, wherein the guide RNA sequence is selected from the group consisting of SEQ ID NOs: 4, 7, 11, 15, 23, 27, 31as well as a sequence encoding a Cas13d and comprising a U6 promotor. AAV vectors have widely been used for gene delivery approaches. However, in contrast to common AAV transduction systems, the AAV vector nucleic acids used in the present invention are one-component systems. Consequently, all elements required for transduction of the target cells and expression of the relevant proteins are comprised by a single vector nucleic acid, thereby facilitating the transduction procedure. In order to further expedite transduction and increase transduction efficacy, the vector nucleic acid is also reduced to a minimal size. Such all-in-one AAV-Cas13-gRNA constructs show superior efficiency and fewer side effects compared to conventional two vector nucleic acid systems. Since AAV2 vectors have shown high transduction capacity in the lung during a Phase III clinical trial, the AAV vectors disclosed herein are preferably based on AAV2 vectors.

The present AAV vectors may comprise either coding sequences for a single Cas13, preferably Cas13d, guide RNA, or a combination of two or more Cas13, preferably Cas13d, guide RNAs as described herein. Using more than one guide RNA may further increase efficacy in targeting and cleaving the virus. In addition, a combination of several guide RNAs may also increase the efficacy and specificity in targeting further mutated viruses, for instance, derivatives of the viruses described herein.

Suitable guide RNA spacer sequences have been designed based on sequence alignments of several human-associated *Coronaviridae* strains (SEQ ID NO:1 to SEQ ID NO:31). Especially taking MERS-CoV into consideration for the design of the guide RNAs, which shows less sequence similarity to the other two closely related coronaviruses SARS-CoV and SARS-CoV-2, allows for the identification of those regions of the viral genome that enable the virus to target human cells. These guide RNA spacer sequences as well as other spacer sequences designed accordingly allow for the specific targeting and cleavage also of *Coronaviridae* strains that might only become clinically relevant in the future.

### Embodiments of the invention

The current outbreak of COVID-19 caused by an infection with the newly identified SARS-CoV-2 has already led to nearly 1.000.000 infections and 50.000 deaths worldwide in only a few months. Although there is apparently an urgent need, an effective drug is to date not available and the development of a vaccine is estimated to take about 12-18 months. Thus, promising new therapy approaches are highly demanded.

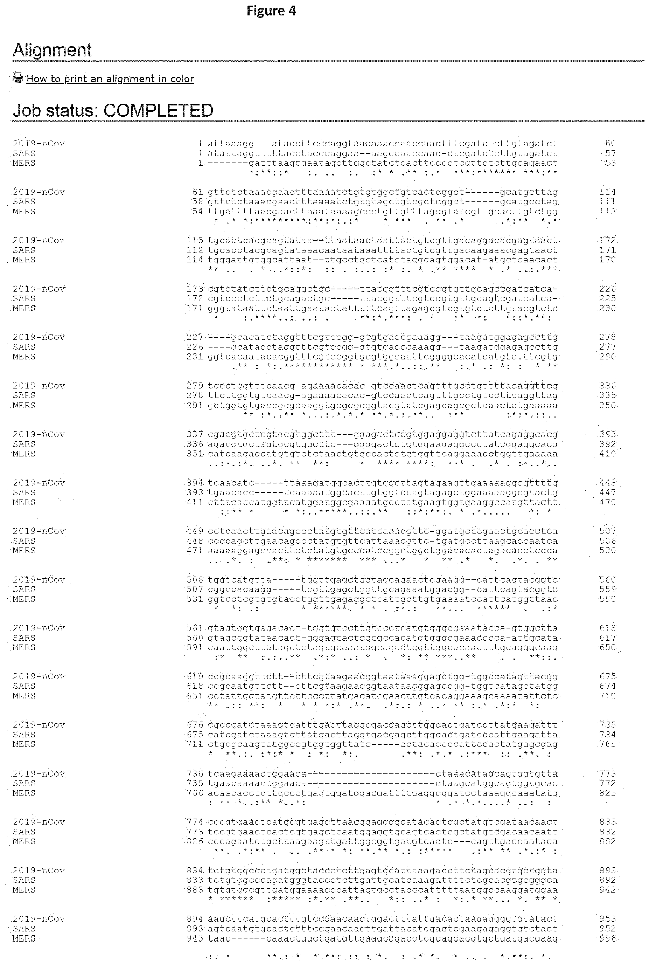

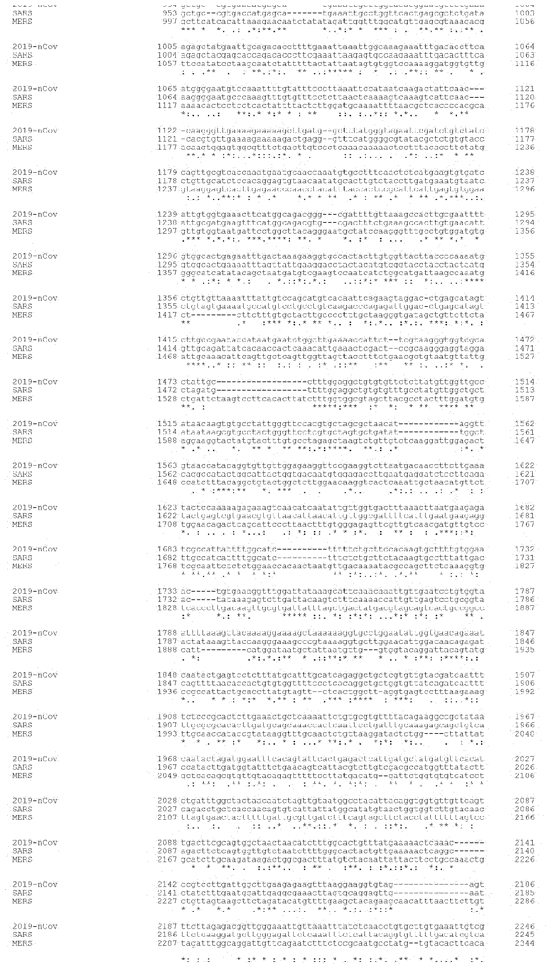

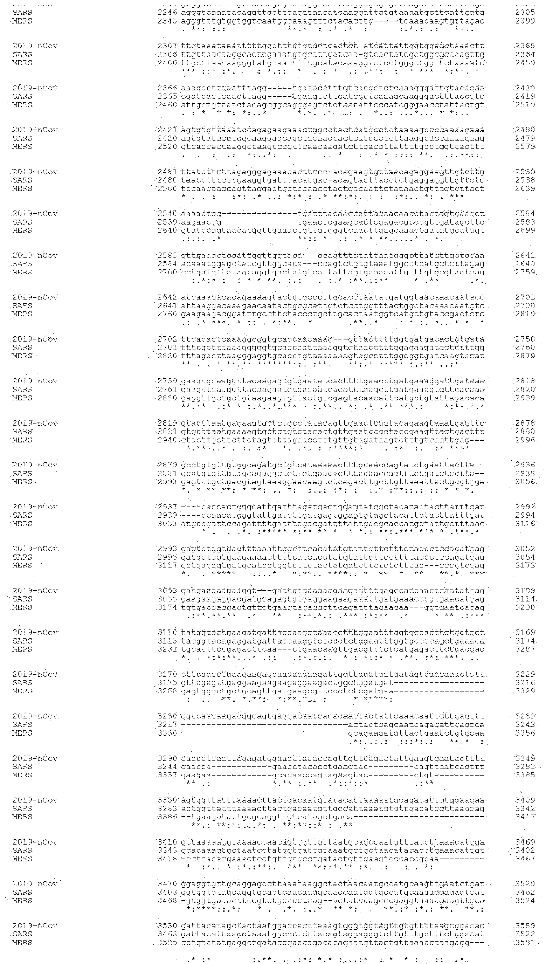

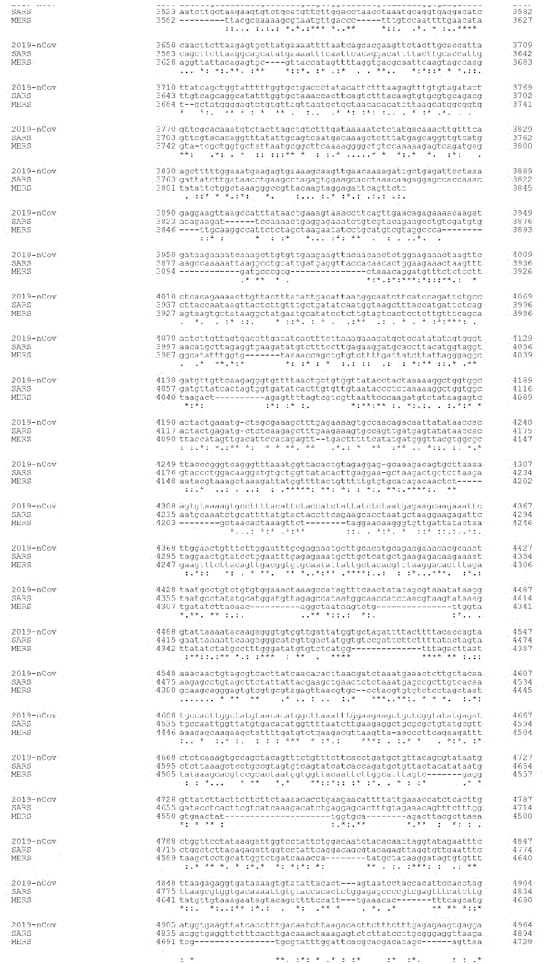

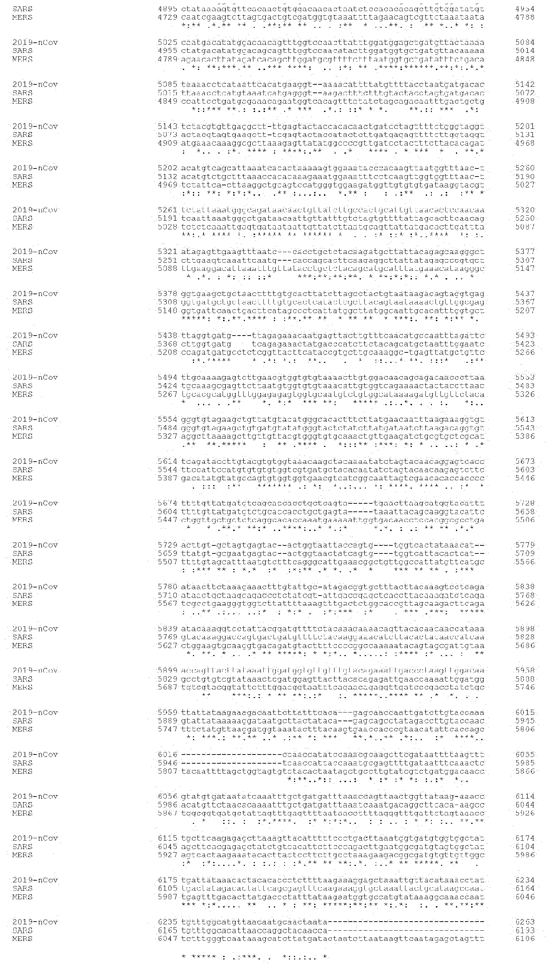

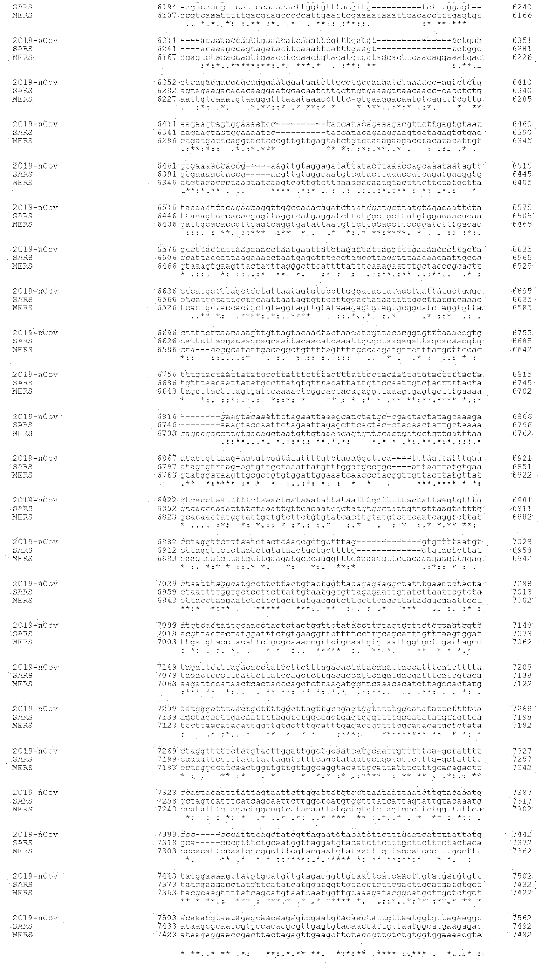

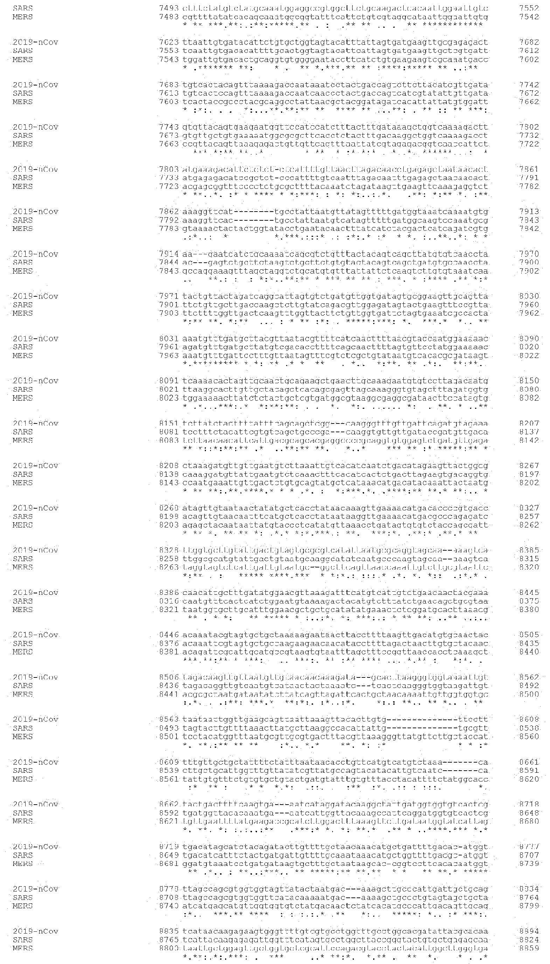

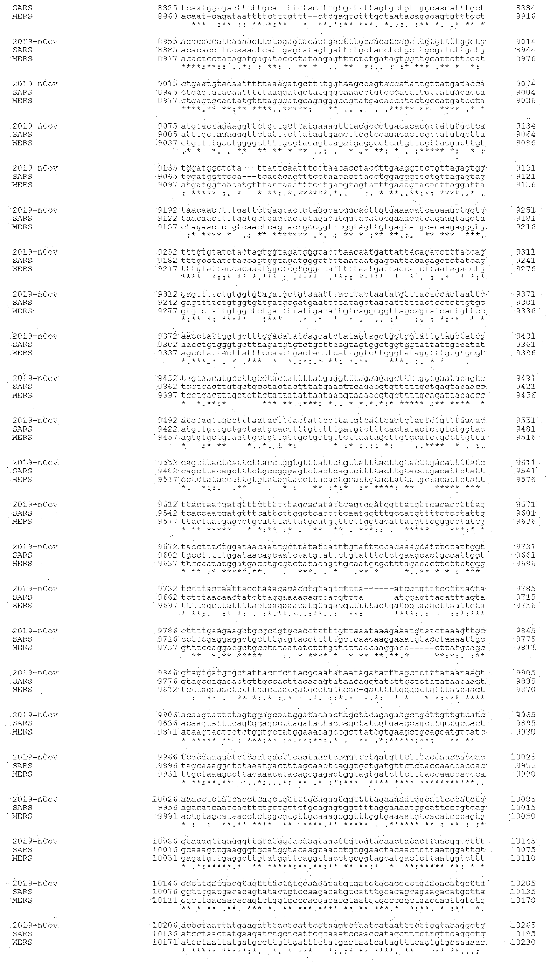

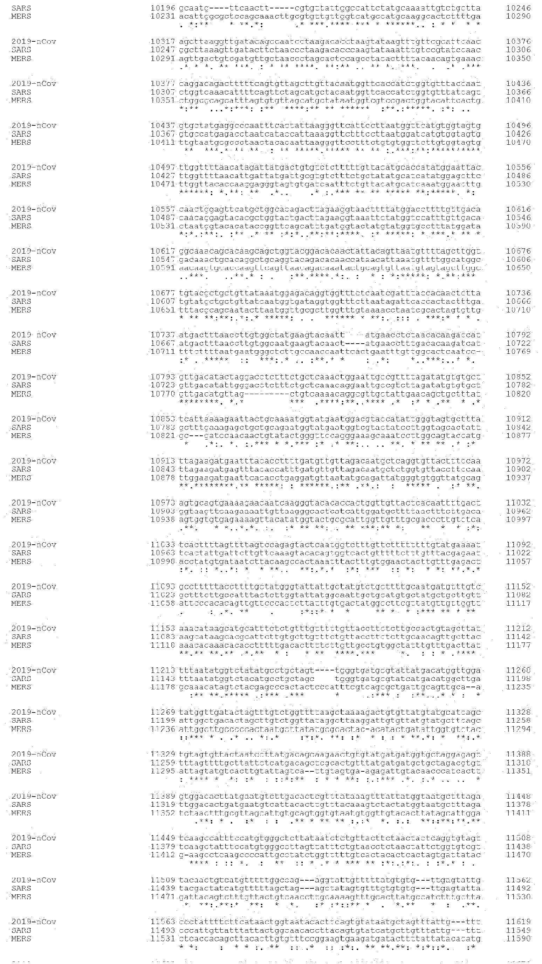

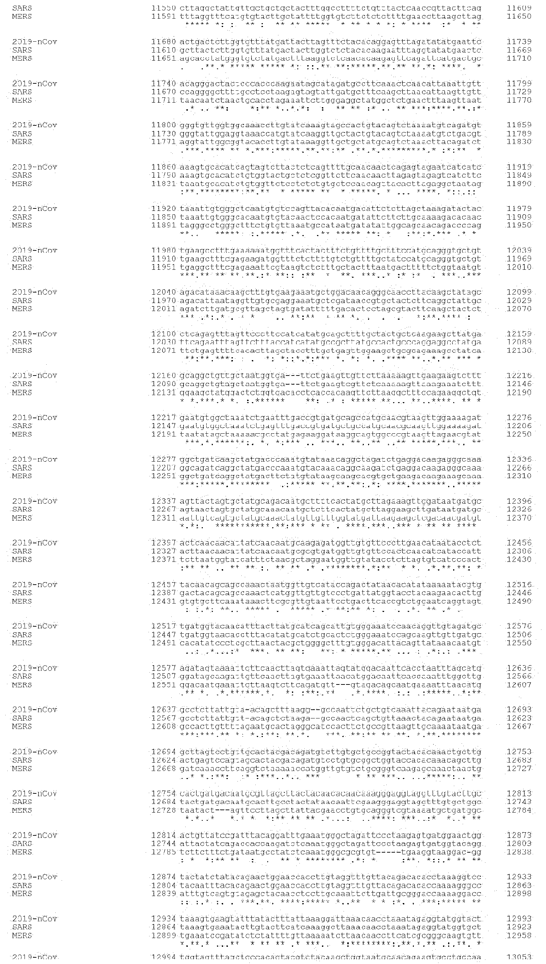

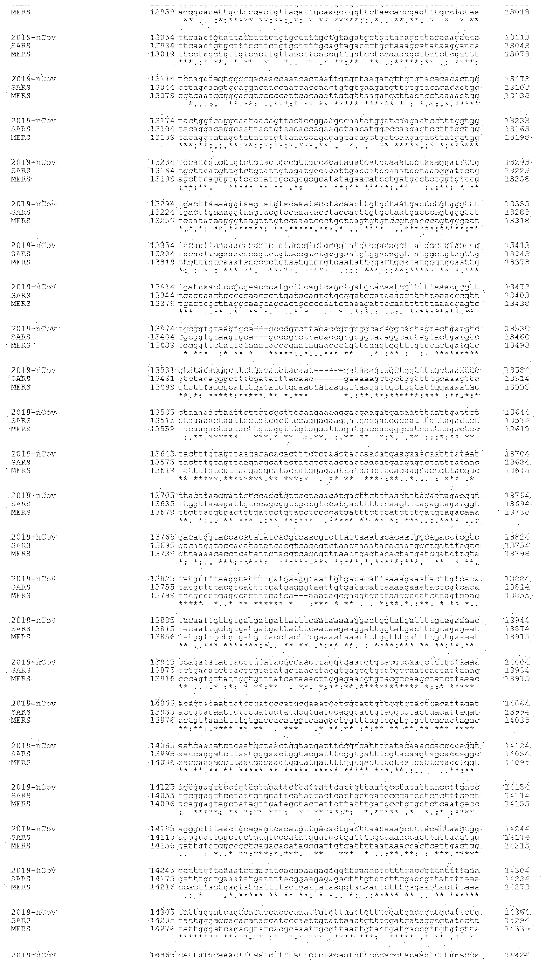

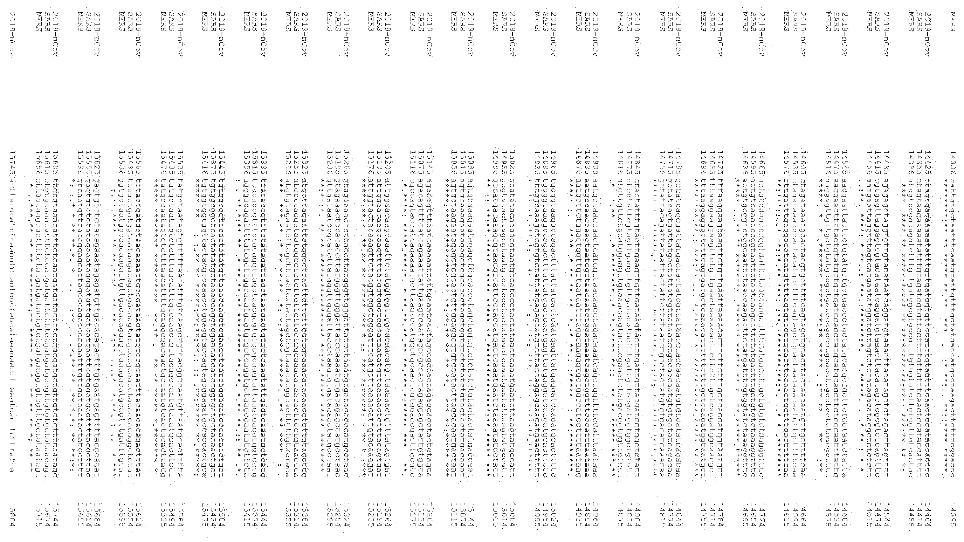

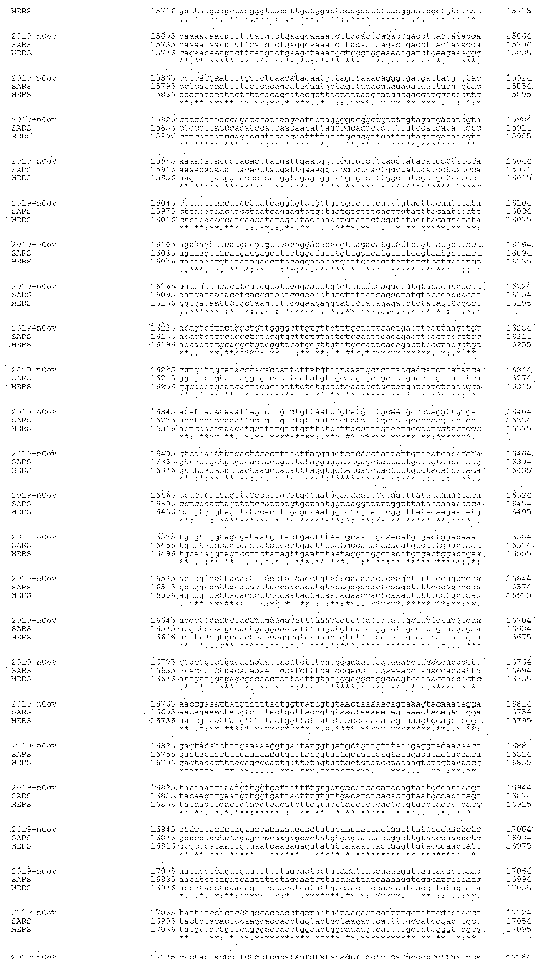

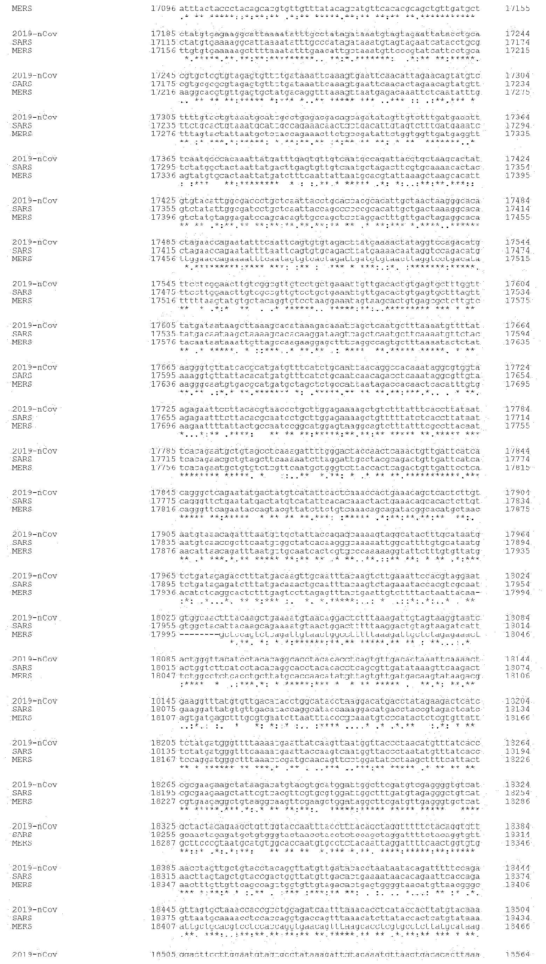

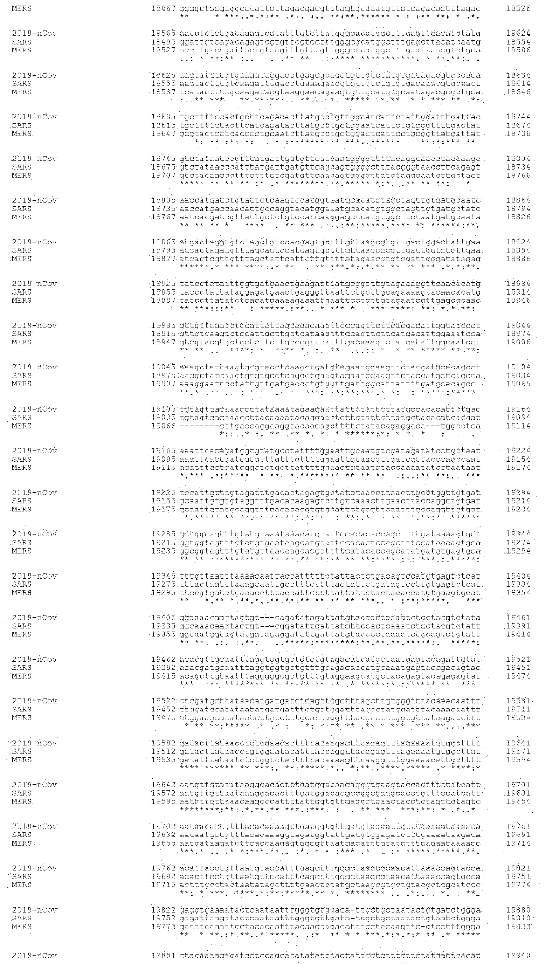

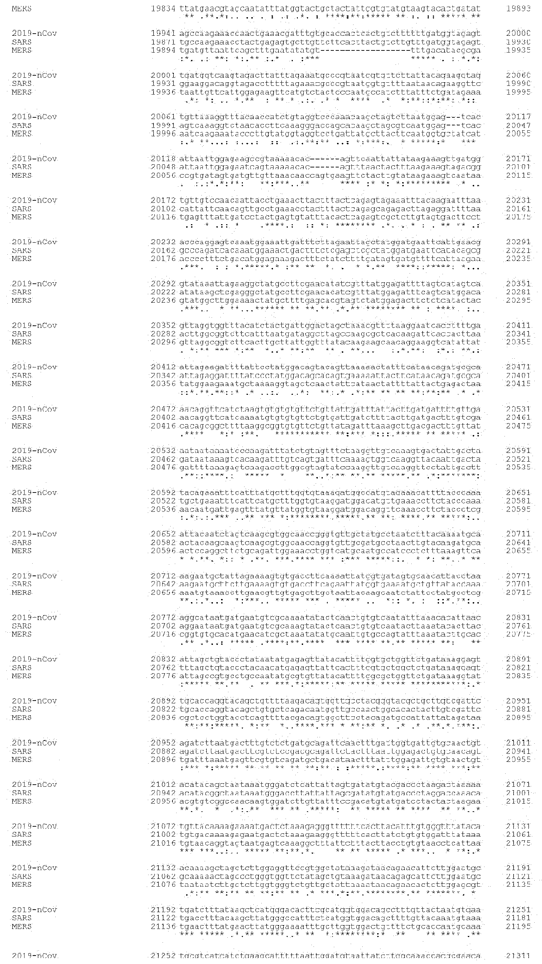

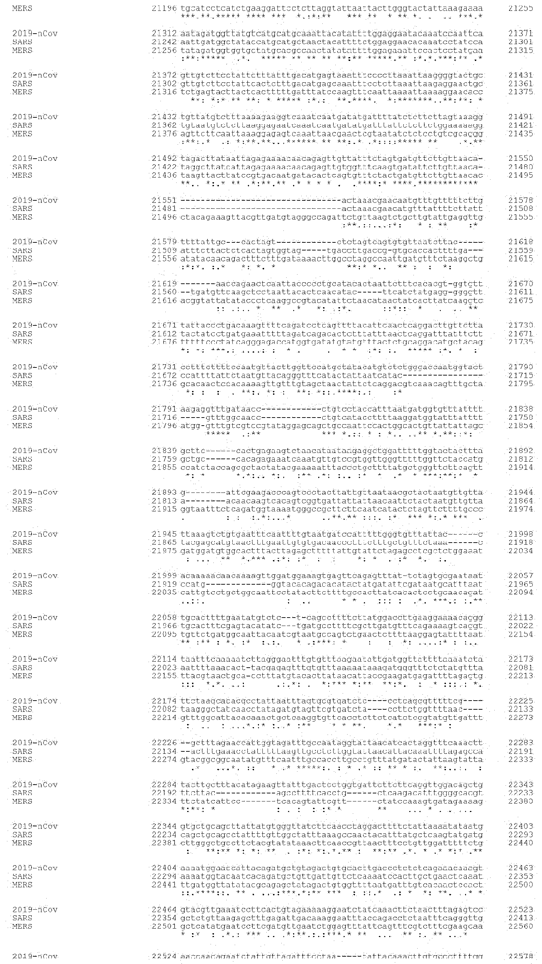

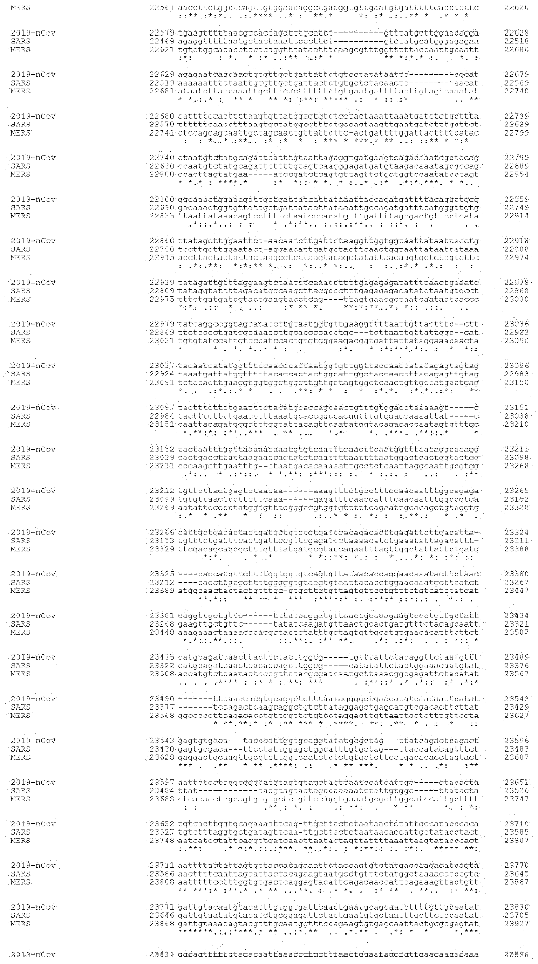

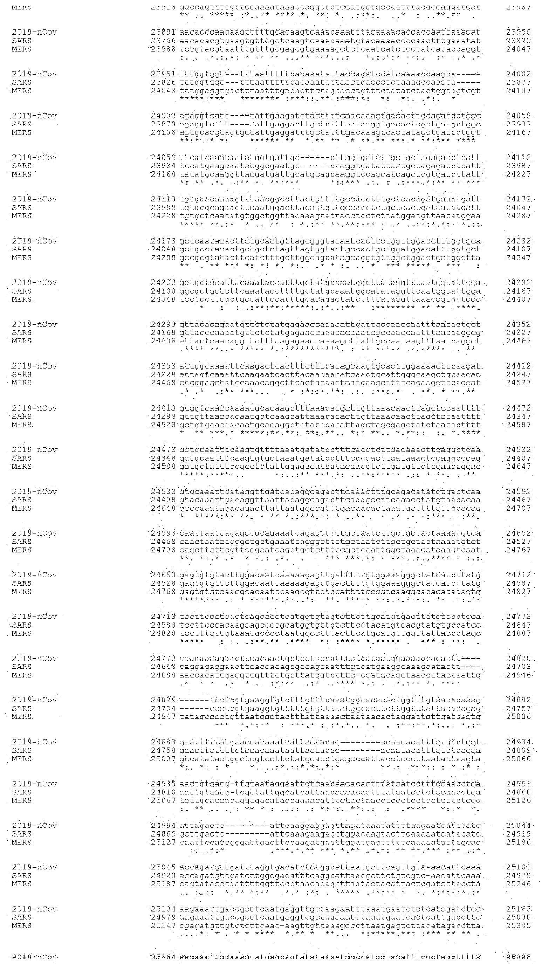

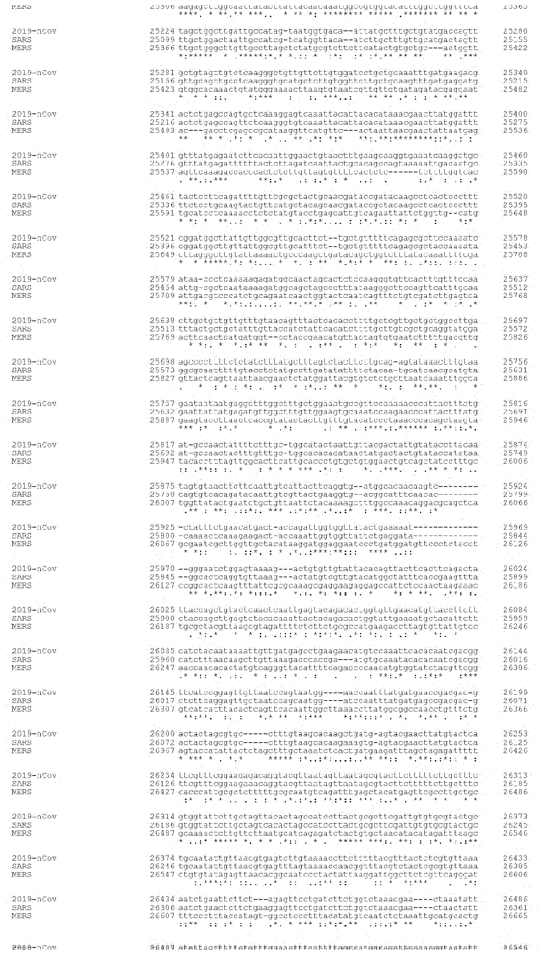

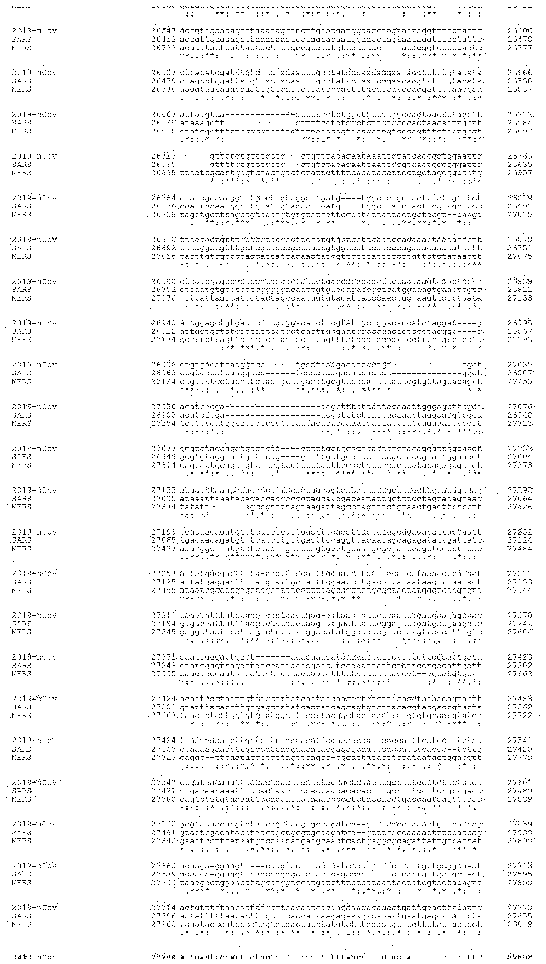

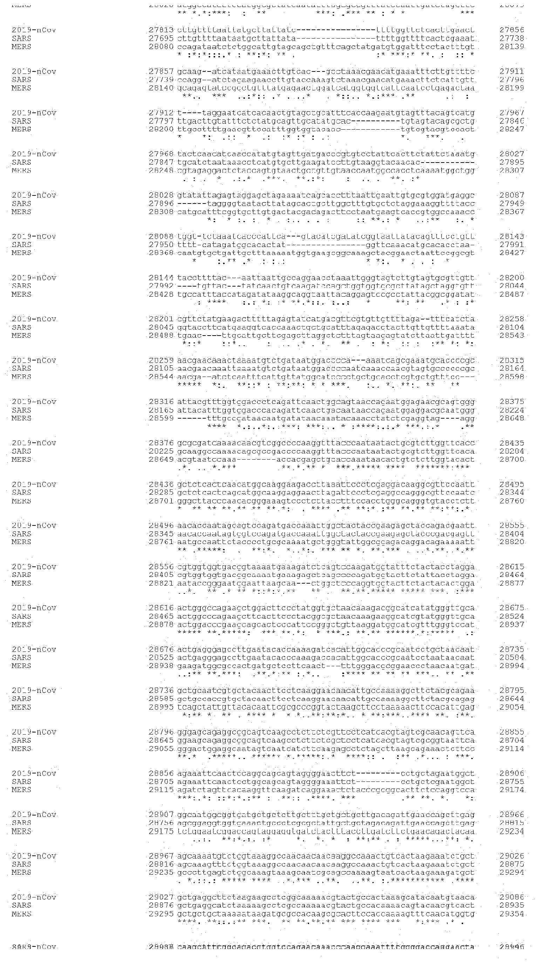

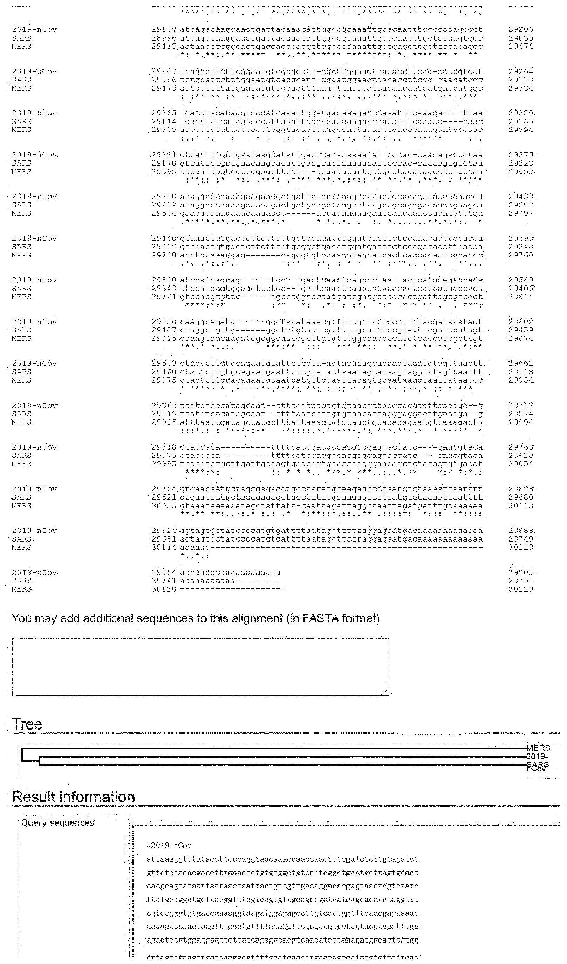

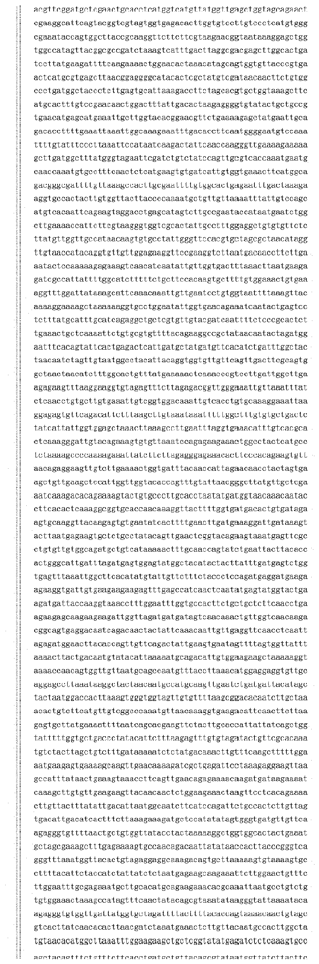

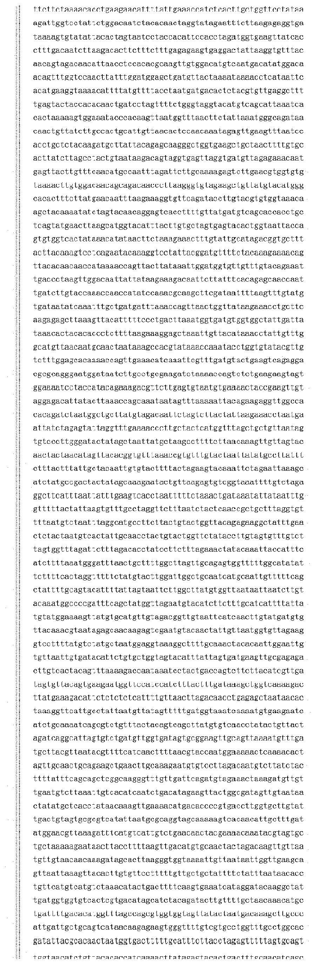

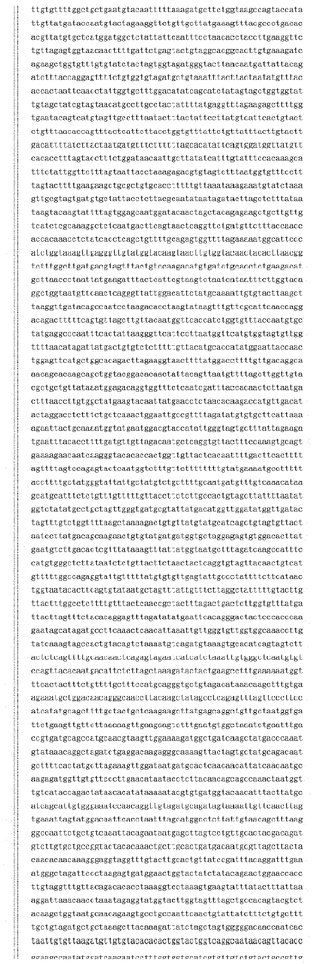

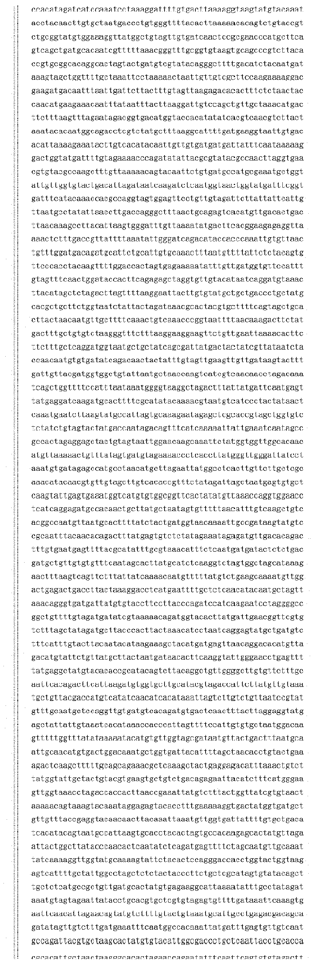

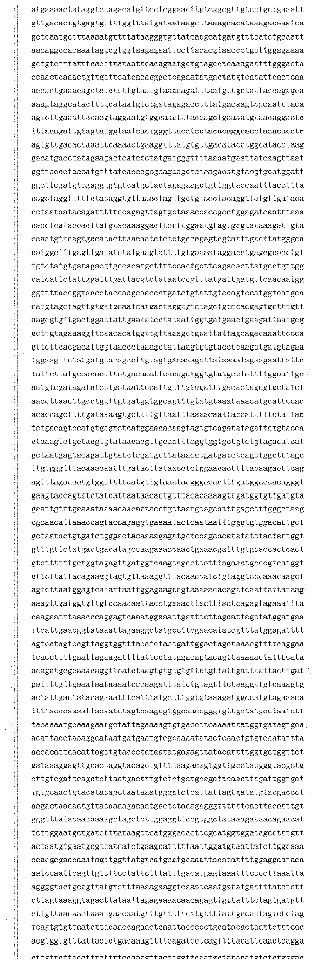

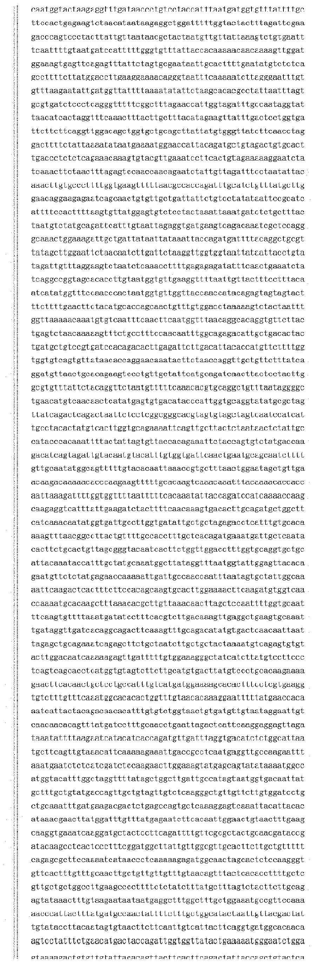

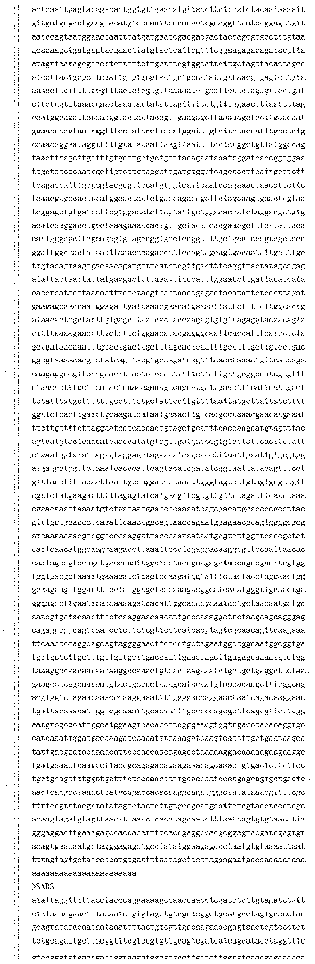

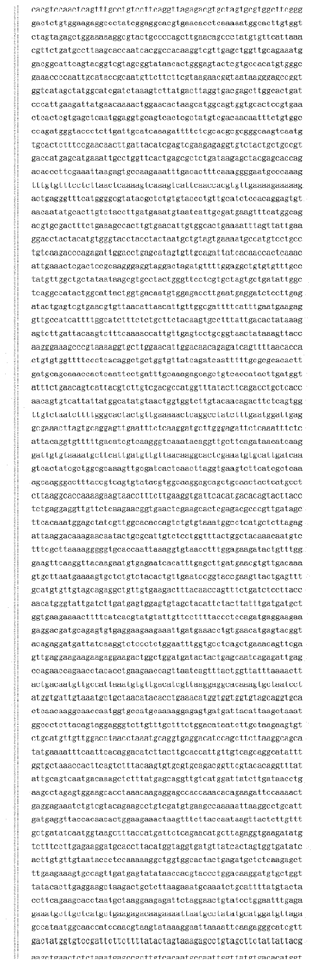

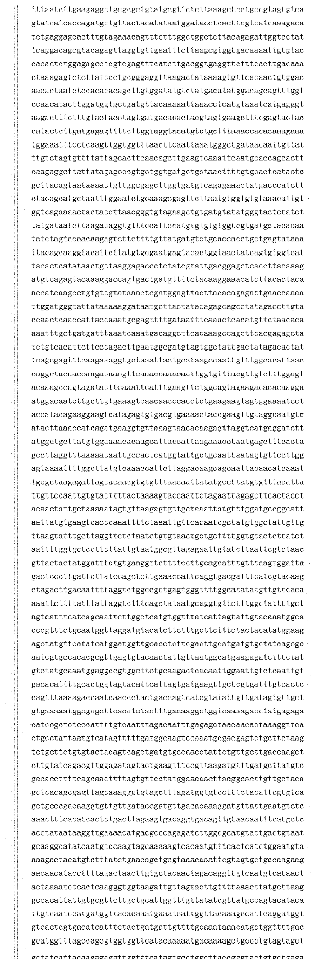

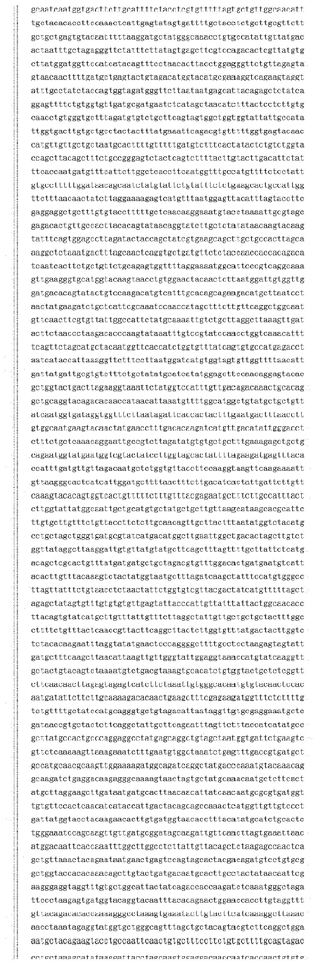

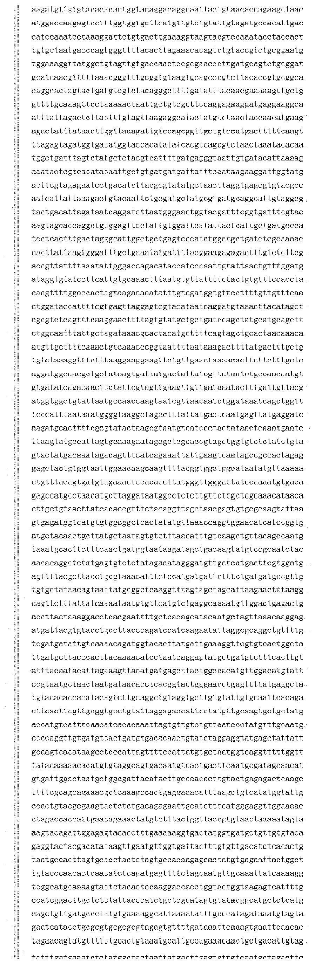

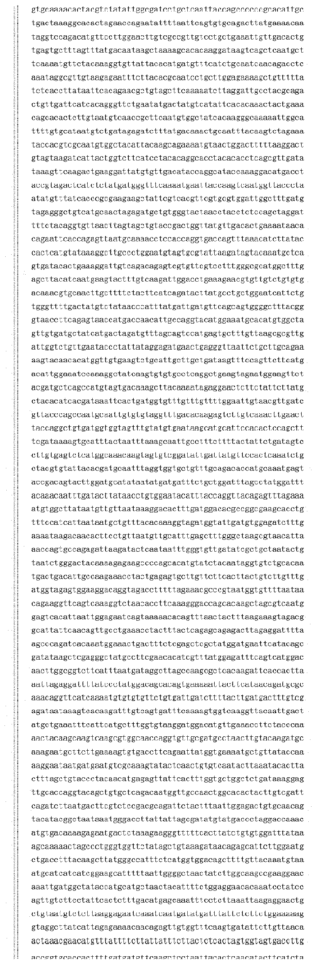

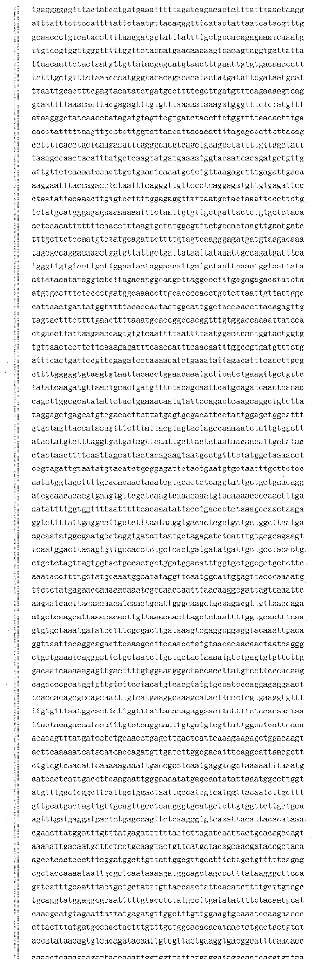

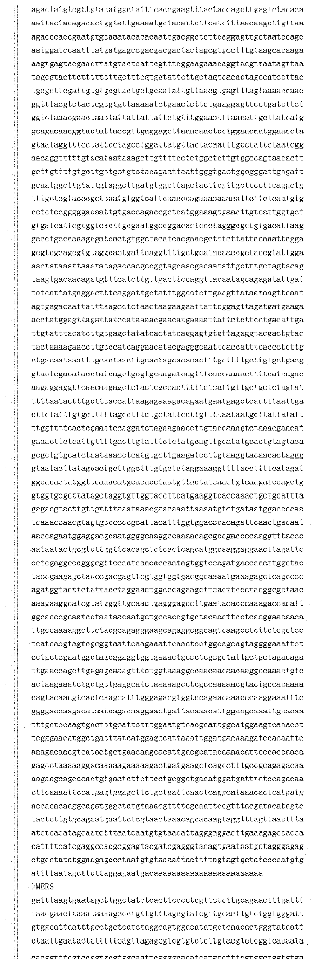

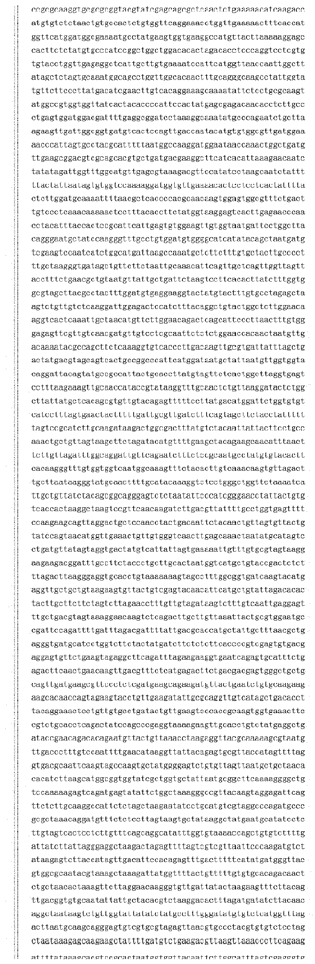

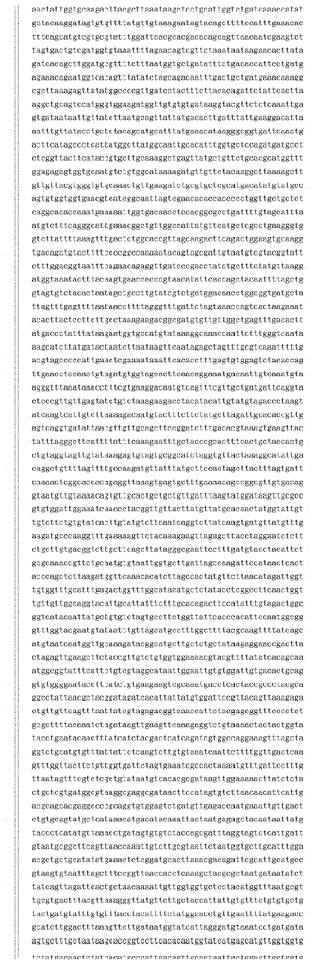

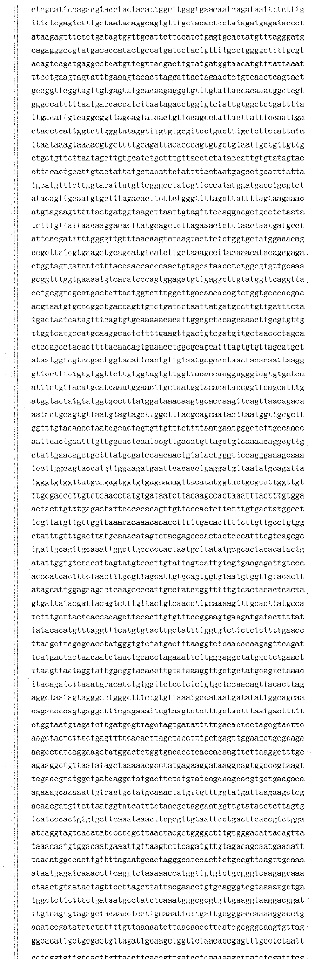

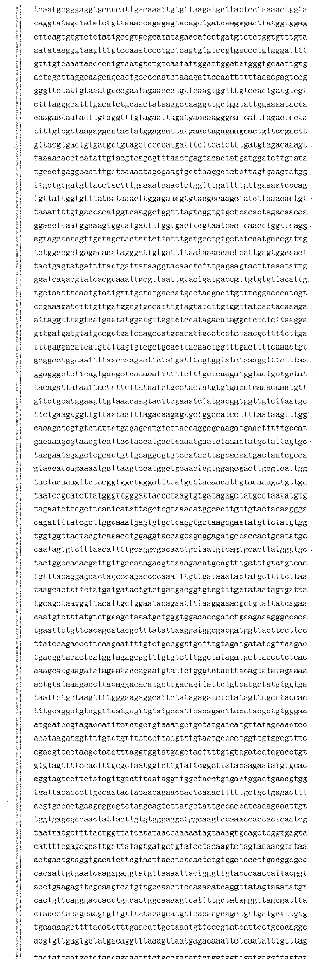

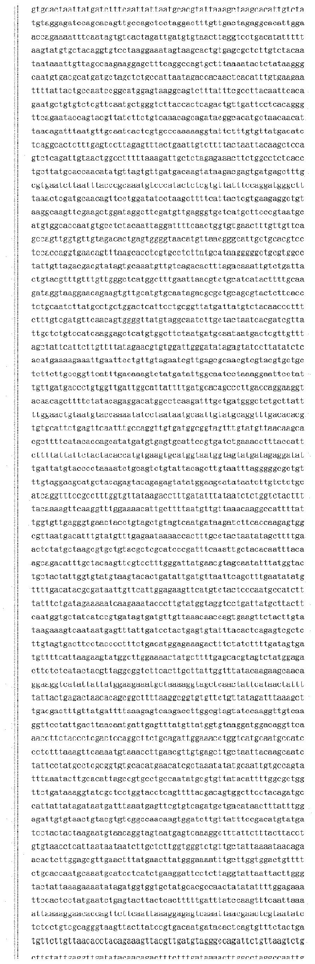

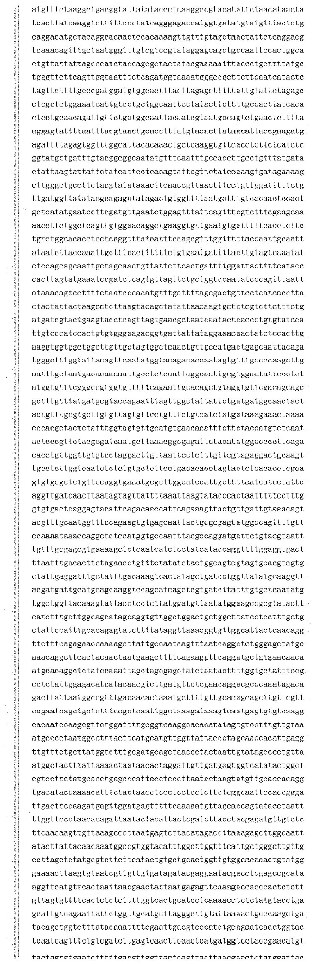

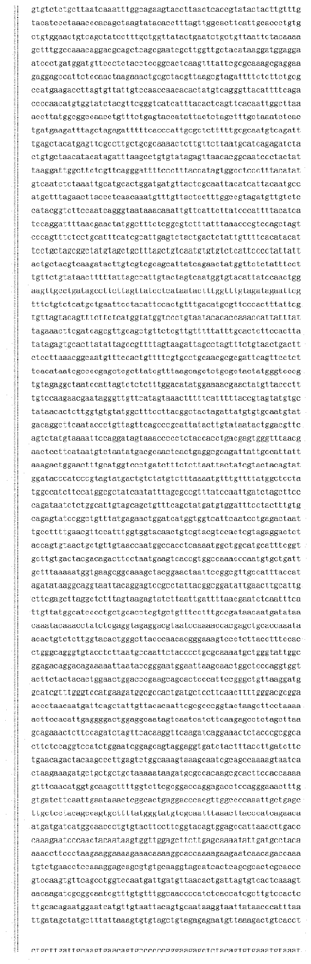

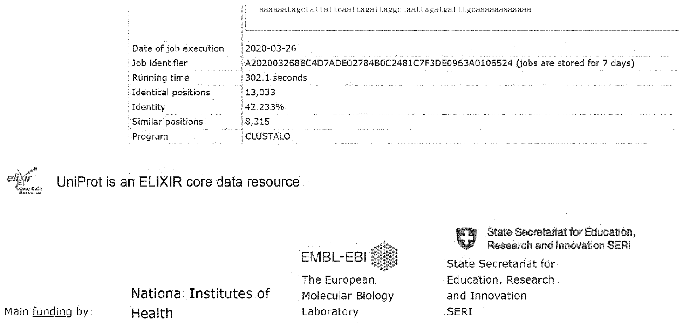

Newly identified SARS-CoV-2 belongs to the family of *Coronaviridae,* a large family of single stranded positive sense RNA viruses. Viruses from the *Coronaviridae* family typically infect the respiratory system and are considered responsible for a number of human illnesses and diseases ranging from the common cold to more severe diseases such as MERS (MERS-CoV) and SARS (SARS-CoV)[1]. SARS-CoV-2 has been reported to contain 10 different proteins (ORF1ab, S, ORF3a, E, M, ORF6, ORF7a, ORF8, N, ORF10) (GenBank entry MN908947.3). A sequence alignment between the genomes derived from MERS-CoV, SARS-CoV and SARS-CoV-2 is shown in Figure 4.

The recently identified and evolved CRISPR/Cas systems have revolutionized gene editing by providing a highly effective, specific and simple system for gene modification in eukaryotic cells. A caspase (Cas) as an effector protein, and a guide RNA for guiding the Cas protein to a specific target sequence within a nucleic acid represent the only required components of a system that allows for the precise cleavage of nucleic acids and/or the genetic modification of cells or even entire organisms [4].

Within the known families of Cas proteins, the Class II, Type VI Cas13 has been recently discovered and classified as four different subtypes: Cas13a, Cas13b, Cas13c and Cas13d [5]. Cas13d is small in size, shows high catalytic activity and specificity in mammalian cells, and targets and cleaves single stranded RNA. Hence, Cas13d offers an exciting new approach for combating viral invasion by degrading single stranded viral RNA.

However, a major obstacle in using Cas13d for combating viral infections is the delivery of the Cas protein and its guide RNA into (infected) cells.

Adeno-associated viruses are non-enveloped, single-stranded DNA viruses of the *Parvoviridae* family. Several serotypes have been identified, among which AAV2 is likely the best known. Adeno-associated viruses exhibit certain characteristics making them an effective gene delivery tool, such as low pathogenicity and low immunogenicity, while being broadly tropic [6].

Thus, the present invention provides a novel therapeutic approach for treating human-associated coronavirus-induced diseases and/or syndromes, particularly COVID-19, by administering to the patient an AAV vector comprising a sequence encoding for a Cas13d protein, as well as a Cas13 guide RNA for targeting specific target sites within the viral genome in order to cleave the target sequence and degrade the virus, wherein the guide RNA sequence comprises a sequence selected from the group consisting of SEQ ID NOs: 4, 7, 11, 15, 23, 27, 31.

. The invention further provides such guide RNAs that are engineered to interact with highly promising target sites within the viral genome.

### Guide RNAs

Cas13 guide RNAs, preferably Cas13d guide RNAs, of the present invention are directed to single stranded RNA target sequences within the genomes of human-associated coronaviruses or derivatives thereof. The target site of the guide RNAs disclosed herein are specifically directed to the conserved sequences and/or sequence portions of several members of the *Coronaviridae* family, preferably between MERS-CoV, SARS-CoV and SARS-CoV-2.

Typical Cas13d guide RNAs target a 22-30 nt target sequence (spacer) [2]. Thus, the guide RNA may be directed to any 22-30 nt target sequence within a coronavirus genome. Typical guide RNAs as used herein are discussed in Konermann et al. [3].

Guide RNA sequences may be designed according to one of the following design approaches:
(1) A sequence alignment of the genome sequences of different strains of coronaviruses, preferably of SARS-CoV-2, SARS-CoV and MERS-CoV, is produced. Spacer sequences of 22-30 nt are designed such that the seed region perfectly matches to regions of 100% overlap between the aligned viral genome sequences. The remainder of the spacer sequence is identical to at least one of the viral sequences, preferably SARS-CoV2, but may only partially match the remaining sequences. Thus, according to this approach, the cutting efficiency is particularly high against all three coronaviruses, while affinity of the guide RNA is maximized for the virus to which the spacer sequence perfectly matches.
(2) A sequence alignment of the genome sequences of different strains of coronaviruses, preferably of SARS-CoV-2, SARS-CoV and MERS-CoV, is produced. Spacer sequences of 22-30 nt are designed such that the seed region perfectly matches at least one of the viral sequences, preferably SARS-CoV-2. In contrast to the first approach, mismatches in the seed region of the spacer sequence to the respective target sequences within some of the viral sequences are tolerated. Guide RNA spacer sequences designed by this approach have very high binding affinities, but have reduced cutting efficiency against those viruses to which the spacer sequence does not perfectly match.
(3) A sequence alignment of the genome sequences of different strains of coronaviruses, preferably of SARS-CoV-2, SARS-CoV and MERS-CoV is produced. Spacer sequences of 22-30 nt are designed such that the guide RNA spacer sequences have the best overall fit for all members of the *Coronaviridae* family that have been subjected to sequence alignment. Based on the requirement that the guide RNA spacer sequences have 100% sequence similarity to respective target sequences of all the aligned coronavirus members in the seed region, an overall sequence similarity of the guide RNA spacer sequences to the respective sequences in the aligned coronavirus members of up to 95 % is achievable. This approach allows for the highest probability of the guide RNAs to also target future coronavirus variants.

Thus, the guide RNA may be directed to a target sequence within conserved regions of genomes of viruses of the *Coronaviridae* family, preferably that of human-associated coronaviruses.

The guide RNA may be directed to a target sequence within conserved regions of genomes of MERS-CoV, SARS-CoV and SARS-CoV-2 or derivatives thereof.

The guide RNA may be directed to a target sequence within the SARS-CoV-2 genome or derivatives thereof.

The guide RNA may comprise a spacer sequence of any of SEQ ID NOs: 1 - 39 or combinations thereof, as shown in Table 1 herein.

The guide RNA may, for example, comprise a spacer sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38 or SEQ ID NO:39, or any combination thereof.

The guide RNA may comprise a spacer sequence of any of SEQ ID NOs: 1-31, or combinations thereof.

The guide RNA spacer sequence may, for example, comprise a spacer sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30 or SEQ ID NO:31, or any combination thereof.

The guide RNA may further comprise a spacer sequence of any of SEQ ID NOs: 3, 4, 11-20, 22, 29 or 31, or combinations thereof.

The guide RNA spacer sequence may for example comprise a spacer sequence of SEQ ID NO:3, SEQ ID NO:4, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:22, SEQ ID NO:29 or SEQ ID NO:31, or combinations thereof.

The guide RNA may comprise a spacer sequence of any of SEQ ID NOs: 3, 4, 11, 12, 19, 20, 22 or 29, or combinations thereof.

The guide RNA spacer sequence may for example comprise a spacer sequence of SEQ ID NO:3, SEQ ID NO:4, SEQ ID NO:11, SEQ ID NO:12, SEQ ID NO:19, SEQ ID NO:20, SEQ ID NO:22 or SEQ ID NO:29, or combinations thereof.

The guide RNA may also comprise a spacer sequence of any of SEQ ID NOs: 13-18 or 31.

The guide RNA may comprise a spacer sequence of any of SEQ ID NOs: 4, 6, 11-16, and 31.

Particularly suitable guide RNAs include the spacer sequences of any of SEQ ID NOs: 4, 7, 15, 23, 27, and 31.

The guide RNA spacer sequence may, for example, comprise a spacer sequence of SEQ ID NO:13, SEQ ID NO:14, SEQ ID NO:15, SEQ ID NO:16, SEQ ID NO:17, SEQ ID NO:18, or SEQ ID NO:31, or combinations thereof.

**Table 1: Guide RNA spacer sequences as disclosed herein**

| **SEQUENCE NO.** | **GUIDE RNA SPACER NAME** | **GUIDE RNA SPACER SEQUENCE** | **APPROACH** |
|---|---|---|---|
| SEQ ID NO:1 | gRNA_XX-O-1 | AACAATTGTATGTGACAAGTATTTCTT | 2 of EX. 2 |
| SEQ ID NO:2 | gRNA_XX-O-2 | TACACGTTCACCTAAGTTGGCGTATAC | 2 of EX. 2 |
| SEQ ID NO:3 | gRNA_XX-O-3 | AGAGAAAGTGTGTCTCTTAACTACAAAG | 1 of EX. 2 |
| SEQ ID NO:4 | gRNA_XX-O-4 | CGGGTTTGACAGTTTGAAAAGCAACATT | 1 of EX. 2 |
| SEQ ID NO:5 | gRNA_XX-O-5 | AATTTGCTTGTTCCAATTACTACAGTA | 2 of EX. 2 |
| SEQ ID NO:6 | gRNA_XX-O-6 | TTAGGATAATCCCAACCCATAAGGTGA | 2 of EX. 2 |
| SEQ ID NO:7 | gRNA_XX-O-7 | TGCATTAACATTGGCCGTGACAGCTTG | 2 of EX. 2 |
| SEQ ID NO:8 | gRNA_XX-O-8 | CTGTGTCAACATCTCTATTTCTATAGA | 2 of EX. 2 |
| SEQ ID NO:9 | gRNA_XX-O-9 | ACTTAAAGTTCTTTATGCTAGCCACTA | 2 of EX. 2 |
| SEQ ID NO:10 | gRNA_XX-O-10 | CATTGAGAAATGTTTACGCAAATATGC | 2 of EX. 2 |
| SEQ ID NO:11 | gRNA_XX-O-11 | AGCTCTATTCTTTGCACTAATGGCATAC | 1 of EX. 2 |
| SEQ ID NO:12 | gRNA_XX-O-12 | ACAAATGTTAAAAACACTATTAGCATAA | 1 of EX. 2 |
| SEQ ID NO:13 | gRNA_XX-O-13 | GAGCTCTATTCTTTGCACTAAT | 3 of EX. 2 |
| SEQ ID NO:14 | gRNA_XX-O-14 | GCATACTTAAGATTCATTTGAGT | 3 of EX. 2 |
| SEQ ID NO:15 | gRNA_XX-O-15 | ACTCTTACCAGTACCAGGTGGTCC | 3 of EX. 2 |
| SEQ ID NO:16 | gRNA_XX-O-16 | CAGCATTACCATCCTGAGCAAAGAA | 3 of EX. 2 |
| SEQ ID NO:17 | gRNA_XX-O-17 | TGTTGGGTATAAGCCAGTAATT | 3 of EX. 2 |
| SEQ ID NO:18 | gRNA_XX-O-18 | GAGCCCTGTGATGAATCAACAGT | 3 of EX. 2 |
| SEQ ID NO:19 | gRNA_XX-S-1 | AAAACACTTGAAATTGCACCAAAATTG | 1 of EX. 2 |
| SEQ ID NO:20 | gRNA_XX-S-2 | AGCCTCAACTTTGTCAAGACGTGAAAG | 1 of EX. 2 |
| SEQ ID NO:21 | gRNA_XX-S-3 | GCCTGTGATCAACCTATCAATTTGCAC | 2 of EX. 2 |
| SEQ ID NO:22 | gRNA_XX-S-4 | TTTGATTGTCCAAGTACACACTCTGAC | 1 of EX. 2 |
| SEQ ID NO:23 | gRNA_XX-E-1 | TAGTGTAACTAGCAAGAATACCACGAA | 2 of EX. 2 |
| SEQ ID NO:24 | gRNA_XX-E-2 | ACGCACACAATCGAAGCGCAGTAAGGA | 2 of EX. 2 |
| SEQ ID NO:25 | gRNA_XX-E-3 | TTTAGACCAGAAGATCAGGAACTCTAG | 2 of EX. 2 |
| SEQ ID NO:26 | gRNA_XX-E-4 | AATACCACGAAAGCAAGAAAAAGAAGT | 2 of EX. 2 |
| SEQ ID NO:27 | gRNA_XX-M-1 | GTAAACAGCAGCAAGCACAAAACAAGC | 2 of EX. 2 |
| SEQ ID NO:28 | gRNA_XX-M-2 | GCTGCGAAGCTCCCAATTTGTAATAAG | 2 of EX. 2 |
| SEQ ID NO:29 | gRNA_XX-N-1 | TGGGGGCAAATTGTGCAATTTGCGGCC | 1 of EX. 2 |
| SEQ ID NO:30 | gRNA_XX-N-2 | TGAGGAACGAGAAGAGGCTTGACTGCC | 2 of EX. 2 |
| SEQ ID NO:31 | gRNA_XX-N-3 | TCAGCAGCAGATTTCTTAGTGA | 3 of EX. 2 |
| SEQ ID NO:32 | gRNA_Q-4-1 | ATATATGTGGTACCATGTCACC | [9] |
| SEQ ID NO:33 | gRNA_Q-4-2 | ATTACCTTCATCAAAATGCCTT | [9] |
| SEQ ID NO:34 | gRNA_Q-4-3 | CTTGATTATCTAATGTCAGTAC | [9] |
| SEQ ID NO:35 | gRNA_Q-4-4 | AAGAATCTACAACAGGAACTCC | [9] |
| SEQ ID NO:36 | gRNA_Q-8-1 | GAAGAGGCTTGACTGCCGCCTC | [9] |
| SEQ ID NO:37 | gRNA_Q-8-2 | GCCTGGAGTTGAATTTCTTGAA | [9] |
| SEQ ID NO:38 | gRNA_Q-8-3 | GTTGTTGTTGGCCTTTACCAGA | [9] |
| SEQ ID NO:39 | gRNA_Q-8-4 | GCCTCAGCAGCAGATTTCTTAG | [9] |

Of the above listed spacer sequences, those referred to as gRNA_XX-O-1 to gRNA_XX-O-18 target the ORF1ab gene, the gRNA_XX-S-1 to gRNA_XX-S-4 sequences target the "S" spike protein gene, the gRNA_XX-E-1 to gRNA_XX-E-4 sequences target the envelope protein gene, the gRNA_XX-M-1 to gRNA_XX-M-2 sequences target the membrane protein gene, and the gRNA_XX-N-1 to gRNA_XX-N-3 target the nucleocapsid protein gene.

### Cas13d

According to the present invention, Cas13, preferably Cas13d, is selected as the endonuclease for cleaving and targeting coronavirus genomes. Due to its small size and high targeting and cleavage efficacy and specificity in mammalian cells, this CRISPR/Cas member appears particularly well-suited for an anti-viral approach.

Cas13d, like the other Cas13 family enzymes, has the property to independently process its own CRISPR arrays into mature guide RNAs that contain a 30 base pair 5' direct repeat followed by a variable 3' spacer that ranges from 22 to 30 bp in length.

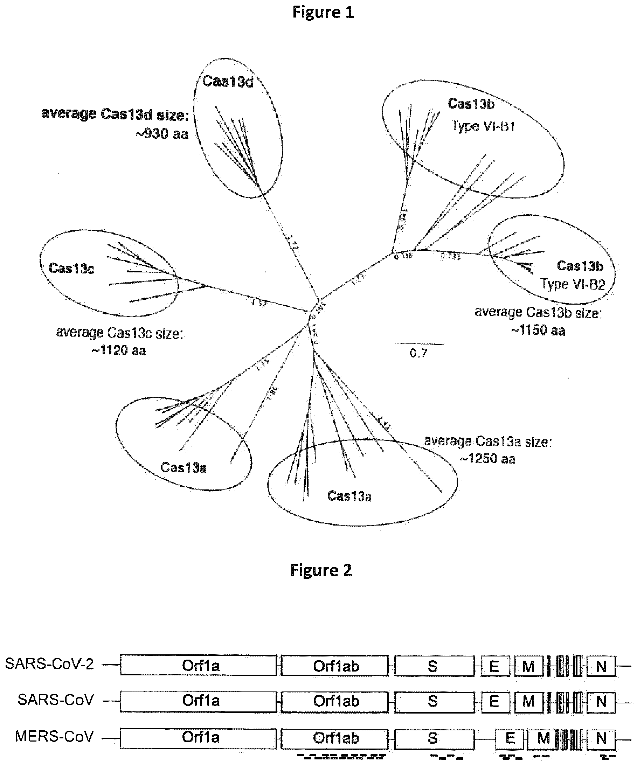

To date, seven different Cas13d proteins have been identified (Figure 1): EsCas13d, RffCas13d, UrCas13d, RaCas13d, P1E0 Cas13d, Adm Cas13d and RfxCas13d. Among these Cas13d variants, RfxCas13d has been reported to show high RNA knock-down efficacy with minimal off-target activity [2].

Thus, in some embodiments, any Cas13d endonuclease may be used.

In preferred embodiments, RfxCas13d endonuclease is used.

### AAV vector comprising a sequence encoding Cas13d and comprising a guide RNA expression cassette

An AAV vector of the present invention comprises a nucleic acid molecule comprising a Cas13d guide RNA expression cassette encoding for at least one guide RNA, wherein the guide RNA sequence is selected from the group consisting of SEQ ID NOs: 4, 7, 11, 15, 23, 27, 31 and a sequence encoding a Cas13d protein and comprising a U6 promotor. The AAV vector serves as a vehicle for the transport of the CRISPR/Cas13d system into a cell, including those infected with a virus.

AAV vectors represent a well-known gene delivery tool suitable for a variety of applications. Dependent on the respective AAV serotype, AAV vectors exhibit remarkable tropism and thus allow for the directed transduction of target cells. For example, AAV9 has proven to show a tropism for myocardial cells and thus, AAV9 based vectors are considered suitable for gene delivery to these cells (see, e.g., EP Patent No. 3 132 041 to Kupatt *et al*.). Similarly, AAV2-based vectors have shown potential for treating cystic fibrosis in humans, as discussed by Guggino *et al.* [7] (see also [8]).

AAV vectors have a packaging limit of only around 4.7 kb, which, for most transduction approaches, entails the use of more than one vector in order to allow for the transfer of all genetic elements required.

However, by selecting the relatively small Cas13d and, where necessary, additionally removing dispensable elements from the AAV vector, the present invention provides a single AAV vector comprising all of the elements required for the expression of Cas13d and its guide RNA in a transduced cell.

Similarly, other Cas13 proteins that do not exceed the packaging size of the AAV vector may be used. Thus, Cas13 proteins having less than 1000 amino acids are well-suited for the vectors according to the present invention.

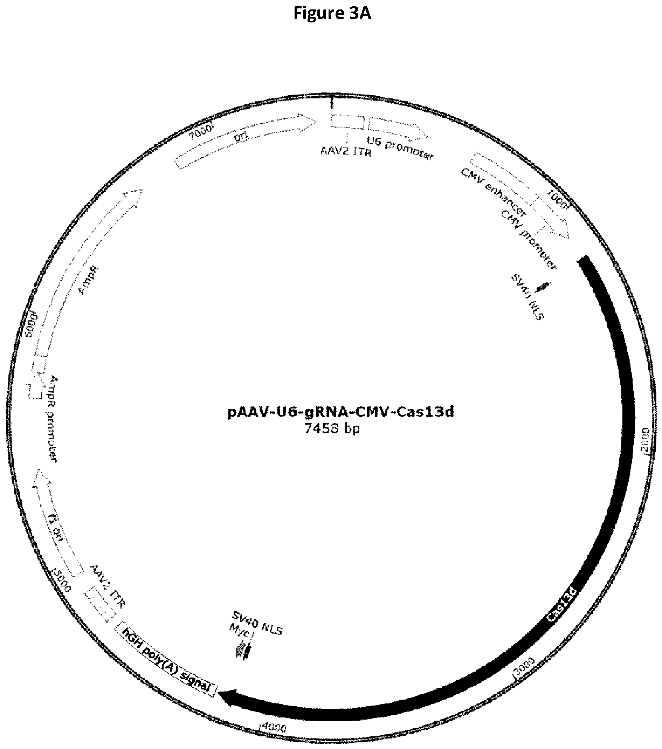

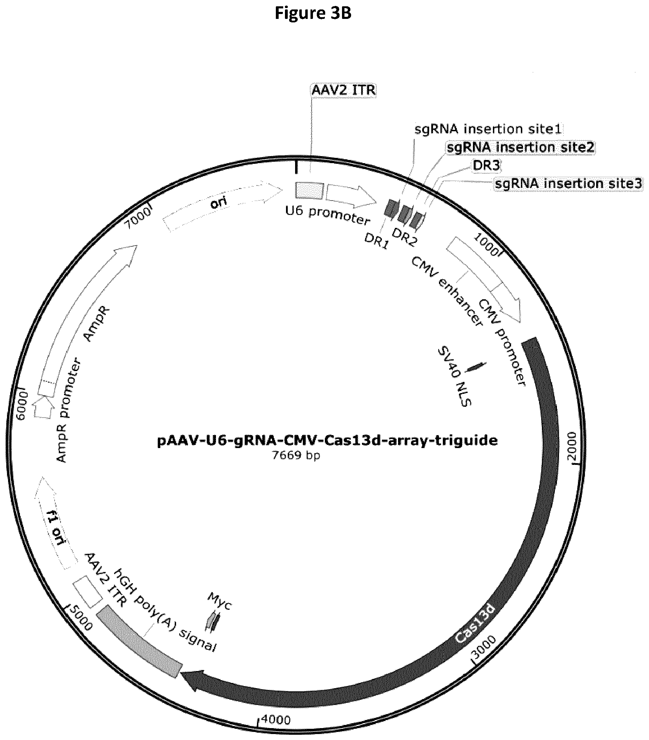

An exemplary schematic map of the AAV2 vector plasmid encoding Cas13d and a guide RNA is depicted in Figure 3A and Figure 3B.

The AAV vector of the present invention may be based on a number of AAV serotypes, including, but not limited to, AAV1, AAV2, AAV3, AAV4, AAV5, AAV6, AAV7, AAV8, or AAV9.

In preferred embodiments, the AAV vector is based on AAV1, AAV2, AAV5 or AAV9.

In even more preferred embodiments, the AAV vector is based on AAV2.

In another even more preferred embodiment, the AAV vector is based on AAV9.

In most preferred embodiments, the AAV vector plasmid is pAAV2-U6-gRNA-CMV-Cas13d (see SEQ ID NO:40).

In another preferred embodiment, the AAV vector plasmid is pAAV2-U6-gRNA-CMV-Cas13d-array-triguide (see SEQ ID NO:41) which allows insertion of three gRNA sequences.

In another preferred embodiment, the AAV vector plasmid is pAAV-U6-gRNA-quadguide-CMV-Cas13d-V3-basic (see SEQ ID NO:42) which allows insertion of four guide RNA sequences. Generation of an AAV vector using the vector plasmid of SEQ ID NO:42 will lead to the packaging of 5064 bp of DNA, having the sequence of SEQ ID NO: 45 into the AAV vector.

It is possible to reduce the size of the AAV vector cargo by not including nuclear localization sequences (NLSs) in the encoded sequence of the Cas13d protein. SARS-CoV-2 proliferation occurs in the cytosol. Thus, by using a Cas13d protein without NLSs, the accumulation of Cas13d in the cytosol can be enhanced, while at the same time the size of the protein and the construct encoding it is reduced. A suitable vector plasmid encoding a Cas13d protein without NLSs is pAAV-U6-gRNA-CMV-Cas13d-Sapl (see SEQ ID NO:43). Generation of an AAV vector using the vector plasmid of SEQ ID NO:43 will lead to the packaging of 4833 bp of DNA, having the sequence of SEQ ID NO: 46 into the AAV vector.

Preferably, the AAV vector contains less than 5kb of DNA. Packaging efficiency and expression in cells drops down significantly at sizes larger than 5 kb [11].

In order to further promote the binding of the Cas13d protein to the SARS-CoV-2 genome, an N-terminal RNA binding domain (N-NTD) can be fused to the Cas13d protein. N-NTD is the RNA-binding domain of the SARS-CoV-2 nucleocapsid (N) protein. The main function of nucleocapsid protein during infection is to bind the viral RNA and form a helical ribonucleoprotein (RNP) complex, in order to protect the viral genome and maintain reliable viral replication. The N-terminal binding domain (N-NTD) of the nucleocapsid protein captures the viral RNA genome and the C-terminal domain anchors the RNP complex to the viral membrane via its interaction with M protein. Thus, a fusion of N-NTD to the Cas13d protein is expected to promote formation of a complex including Cas13d and the viral genome, and thus to guide the Cas13d into spatial proximity with the viral genome. A suitable vector plasmid encoding a Cas13d protein with N-NTD fused to its C-terminus is pAAV-U6-gRNA-CMV-Cas13d-NTD-Aarl (see SEQ ID NO:44). Generation of an AAV vector using the vector plasmid of SEQ ID NO:44 will lead to the packaging of 5238 bp of DNA, having the sequence of SEQ ID NO: 47 into the AAV vector.

Depending on which respective serotype the AAV vector of the instant invention is based on, the AAV vector may show a tropism for certain cell types, tissues and organs harboring these cell types.

Thus, the AAV vector of the present invention may be directed to various cell types and organs in the human body by selecting a specific serotype of the AAV vector.

In preferred embodiments, the AAV vector shows a tropism for, and is directed to, cells of the human respiratory system.

In even more preferred embodiments, the AAV vector is an AAV2 vector directed to cells of the human respiratory system.

In another preferred embodiment, the AAV vector is directed to human myocardial cells.

In even more preferred embodiments, the AAV vector is an AAV9 vector directed to cells of the human myocardium.

AAV vectors of the present invention may encode a single Cas13d guide RNA or a combination of several guide RNAs. Thus, an AAV vector may encode 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 or 39 guide RNAs.

In preferred embodiments, the AAV vector encodes a single guide RNA.

In another preferred embodiment, the AAV vector encodes two guide RNAs.

In the most preferred embodiments, the AAV vector encodes three, four, or five guide RNAs. Preferably, the spacer sequences of the guide RNAs target different genes of the SARS-CoV-2 genome.

Suitable combinations of guide RNAs for use in the present invention have the spacer sequences of SEQ ID NOs: 4, 7, and 15; SEQ ID NOs: 15, 23, and 31; SEQ ID NOs: 15, 27, and 31; SEQ ID NOs: 23, 27, and 31; SEQ ID NOs: 4, 15, 23, 27, and 31; SEQ ID NOs: 4, 7, 27, and 31; SEQ ID NOs: 4, 23, and 31; SEQ ID NOs: 7, 27, and 31; SEQ ID NOs: 4, 7, 15, 23, 27, and 31.

Particularly preferred guide RNAs for use in the present invention have the spacer sequences of SEQ ID NOs: 4, 7, and 15; SEQ ID NOs: 4, 15, 23, 27, and 31; and SEQ ID NOs: 4, 7, 27, and 31.

In preferred embodiments, the AAV vector encodes the combination of guide RNAs under the same promoter.

### AAV vectors encoding Cas13d and Cas13d guide RNA for treating viral infections

The present invention provides a novel therapeutic approach for treating human-associated coronavirus infections. By introducing an AAV vector into cells, and upon Cas13 expression, the Cas13 is guided by a guide RNA to a target sequence within the viral genome, for cleavage and thus disruption to the genomic sequence. In cells already infected cells Cas13 with a coronavirus, the viral sequence is cleaved leading to viral degradation and inhibition of any additional spread of viral contamination. In cells not yet infected, expression of the Cas13 guide RNA system offers a protective mechanism to such cells by immediately degrading the viral genetic material upon entry into the cell.

In order to reach a target cell, the AAV vector is delivered into tissues and organs harboring the target cells.

According to current reports, coronavirus-caused diseases mainly manifest in the respiratory system of the infected subjects, leading to mild to severe respiratory symptoms and reactions. In particular, the MERS, SARS, and COVID-19 corona variants commonly lead to lung inflammation, pulmonary distress, and acute respiratory syndrome, often caused or driven by cytokine storms of the overpowering immune system. Other scientific publications report findings suggesting that at least the COVID-19 variant may also adversely affect the myocardium in some patients. Consequently, during patient treatment, the AAV vector is delivered to the respiratory system, in particular the lungs, and/or the myocardium as indicated.

Thus, in one embodiment, the AAV vector for treating human-associated coronavirus-induced disease is administered to the respiratory system of the patient.

In another embodiment, the AAV vector is administered to the lower respiratory tract of the patient.

In a preferred embodiment, the AAV vector is administered to the upper respiratory tract by means of inhalation.

The AAV vector may be administered directly to the trachea tissue.

In an embodiment of specifically treating MERS, SARS, and/or COVID-19, the AAV vector may be administered to the upper respiratory tract of the patient.

In another embodiment of treating MERS, SARS, and/or COVID-19, the AAV vector may be administered to the lower respiratory tract of the patient.

The AAV vector comprising the guide RNA expression cassette and encoding the Cas13d sequence may be comprised by a composition.

In an embodiment, the composition may be in the form of a tablet, capsule, syrup, film, liquid, solution, powder, paste, aerosol, injection, cream, gel, lotion or drops.

In another embodiment, the composition is in the form of an aerosol and administered through an inhaler, nebulizer or vaporizer.

In preferred embodiments, the composition is in the form of an aerosol and administered through an inhaler.

In an even more preferred embodiment, the composition is in the form of an aerosol and administered through an inhaler to the upper respiratory tract.

In another preferred embodiment, AAV vector or a composition comprising same is administered to the patient through a ventilator.

### Adenoviral vectors

All aspects of the present invention can also be practiced with adenoviral vectors instead of AAV vectors. Adenoviral vectors have the advantage of a higher packaging size limitation.

Accordingly, an adenoviral vector may comprise a sequence encoding Cas13d and a guide RNA expression cassette. The adenoviral vector may be delivered to target cells and may be used to treat human-associated coronavirus infections, such as a SARS-CoV-2 infection.

### Delivery of guide RNAs and Cas13d protein independently of viral vectors

Guide RNAs of the present invention can also be delivered to target cells without the use of viral vectors. For instance, the method described in reference [10] may be used, involving the delivery of Cas13 mRNA generated by *in vitro* transcription concomitantly with synthetic guide RNAs. A molar ratio of guide RNA to mRNA of 50:1 may be used. The mRNA and guide RNAs may be delivered by means of vesicles.

The present invention also provides for the AAV vector, the adenoviral vector or the composition of the invention for use in treating human-associated coronavirus infections, such as a SARS-CoV-2 infection, by the delivery of synthetic guide RNAs and an mRNA encoding a Cas13 protein to the respiratory tract of a patient in need thereof. Details of this method are described in reference [10]. The Cas13 protein may be a Cas13a protein. The Cas13 protein may be a Cas13d protein. The mRNA and guide RNAs may be delivered by means of vesicles.

### EXAMPLES

### Example 1

The use of the AAV vectors disclosed herein represents a promising new therapeutic approach for the prevention and/or treatment of coronavirus-induced diseases and syndromes. Therefore, a number of clinical trials are presently ongoing to test the suitability of these vector vehicles in treating a variety of diseases. Clinical trials as well as the respective AAV vector tested are indicated in Table 2 below.

**Table 2: AAV vectors involved in clinical trials**

| **Clinical Trial No.** | **Subject** |
|---|---|
| NCT00976352 | AAV1 Vector Carrying Wildtype GAA Gene for Treating Pompe Disease |
| NCT00749957; | AAV2 Vector Carrying Wildtype RPE65 Gene for Treating Leber Congenital Amaurosis; |
| NCT03496012 | |
| | AAV Vector Carrying Wildtype REP1 Gene for Treating Choroideremia |
| NCT03489291 | AAV5 Vector Carrying Padua Variant of FIX cDNA for Treating Hemophilia A |
| NCT03362502 | AAV9 Vector Carrying a Truncated Human Dystrophin Gene (Mini-Dystrophin) for Treating Duchenne Muscular Dystrophy |

### Example 2 - Design of guide RNA sequences

In order to create an AAV vector encoding a Cas13d and a guide RNA for treating human-associated coronavirus-induced disease, guide RNA spacer sequences were designed.

Similar to the cases involving SARS-CoV and MERS-CoV, the SARS-CoV-2, the pathogen that causes COVID-19, has successfully survived the transmission from animal to human. Based on this observation, the inventors considered that there should be highly conserved regions existing between the genomes of SARS-CoV, MERS-CoV and SARS-CoV-2 that are responsible for the high pathogenicity and infectious properties of these viruses in humans. The inventors also concluded that such conserved genomic regions offer a promising target site for CRISPR/Cas-mediated cleavage, involving a guide RNA that guides a Cas13 protein, preferably a Cas13d protein, to its target and cleavage site to thereby cleave and degrade the viral nucleic acid.

Thus, a sequence alignment of the nucleic acid sequences derived from the SARS-CoV, MERS-CoV and SARS-CoV-2 coronavirus variants was created and five highly conserved regions were identified: ORF1ab, S, E, M and N (see Figure 4). Thirty-one guide RNA spacer sequences were identified targeting the conserved regions and represent highly promising targets for pathogen cleavage and degradation (SEQ ID NO:1 to SEQ ID NO:31).

In order to identify similar sequences between all three SARS-CoV-2, SARS-CoV and MERS-CoV variants, three main approaches are used:
(1) In a first approach, spacer sequences are identified that comprise a seed sequence containing a 7 base pair region precisely overlapping between the sequences of all three virus genomes. This sequence was included as the seed region (15^{th}-21^{st} base of a guide RNA spacer sequence; [2]) of the guide RNA spacer sequences. The remainder of this spacer sequence perfectly matches the SARS-CoV-2 sequence, but only partially matches the MERS-CoV and SARS-CoV variants. The seed region is considered the critical sequence for the targeting specificity of a guide RNA of Cas13d. Consequently, in case of a mismatch of the critical seed sequence with the target sequence, Cas13d cannot cleave the target sequence. Thus, with this approach, the best cutting efficiency is achieved against all three coronaviruses, while affinity of the guide RNA is maximized for SARS-CoV-2.
(2) In a second approach, by increasing sequence similarity between the guide RNA spacer sequence and the respective target sequence over the entire length of the spacer sequence, the binding affinity of the spacer sequence to the viral RNA is increased. Different from the first approach, mismatches in the seed region of the spacer sequence to respective target sequences within SARS-CoV and MERS-CoV have been tolerated. However a 100% sequence similarity between the seed region sequence and a respective target sequence in SARS-CoV-2 is given. Guide RNA spacer sequences designed by this approach have very high binding affinities, but show reduced cutting efficiency against MERS-CoV and SARS-CoV.
(3) In a third approach, guide RNAs are identified that afford the best overall fit for all three members of the coronavirus family. Based on the requirement that the guide RNA spacer sequences have 100% sequence similarity to respective target sequences of all three coronavirus members in the seed region, an overall sequence similarity of the guide RNA spacer sequences to respective sequences in the three coronavirus members of up to 95 % was achieved. This approach allows for the highest probability of the guide RNAs to also target future coronavirus variants.

The above identified guide RNA spacer sequences have been aligned with all human-associated viral transcripts of SARS-CoV-2 known to date. Spacer sequences showing a high target sequence specificity, and thus reducing the risk of potential therapy-induced side effects, have been selected therefrom and each inserted into a pAAV-U6-gRNA-CMV-Cas13d plasmid comprising *inter alia* a U6 promoter for the guide RNA expression cassette as well as a sequence encoding the Cas13d protein (see also SEQ ID NO:1 to SEQ ID NO:40).

### Example 3 - Evaluation of efficiency of single guide RNA sequences

The inhibitory potency of the 39 gRNAs of Table 1 was assessed in a luciferase assay by the co-transfection of pMir-reporter and all-in-one Cas13 guide constructs. A guide RNA targeting LacZ was used as the negative control.

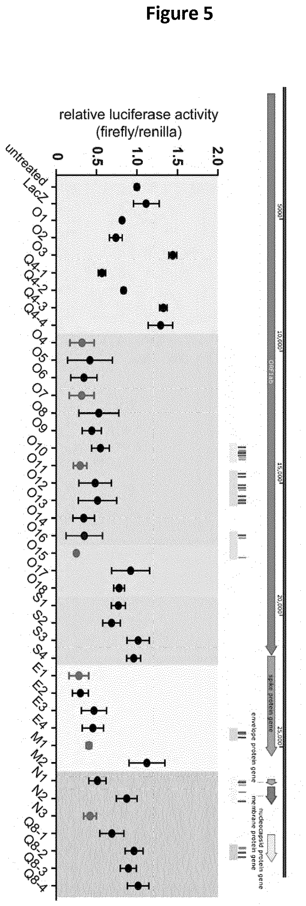

The first screening experiments were performed with non-infectious materials. The 39 guide RNAs (gRNAs) target 6 different regions: gRNA_XX-O-1 to gRNA_XX-O-18 target the ORF1ab gene, the gRNA_XX-S-1 to gRNA_XX-S-4 sequences target the "S" spike protein gene, the gRNA_XX-E-1 to gRNA_XX-E-4 sequences target the envelope protein gene, the gRNA_XX-M-1 to gRNA_XX-M-2 sequences target the membrane protein gene, and the gRNA_XX-N-1 to gRNA_XX-N-3 target the nucleocapsid protein gene. Thus, we cloned the 6 regions by PCR and inserted them into the pMIR vector (a 3'-UTR luciferase vector). Hereby 6 luciferase constructs designated as pMIR-report-SARS-COV-2-fragment 1-6 were obtained. To assess the inhibitory potency of each gRNA, human embryonic kidney (HEK293) cells were co-transfected with the all-in-one Cas13 guide construct and its corresponding pMIR reporter construct. The *LacZ* guide was used as the negative control. Out of 39 gRNAs, 7 guides were selected in the end to make further experiments (highlighted in red in Fig. 5).

### Example 4 - Testing the Cas13-guide RNA system in SARS-CoV-2-infected human epithelial lungs cells

### Experimental design 1

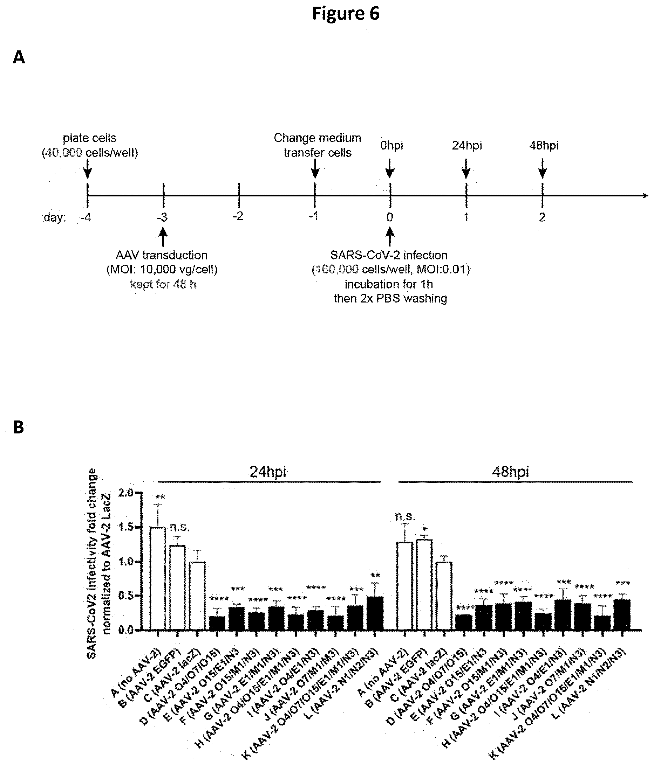

To assess the inhibitory effect of our system, we first packed each of several guide RNA constructs into AAV2 viruses. Each AAV was applied with a titer of 10,000 vg/cell (viral genomes per cell) to transduce human bronchial epithelial Calu-3 cells (40,000 cells/well), which is a typical cell line for coronavirus *in vitro* studies, with different combinations of guide RNA constructs. After 72 hours, the AAV-transduced cells were further infected by SARS-CoV-2 virus with MOI (multiplicity of infection - refers to the number of viral particles per cell) of 0.01. 1 hour after incubation at 37°C, the infectious medium was removed and cells were washed 2x with DPBS. Afterwards, culture medium was collected at time points of 24 hpi (hours post infection) and 48 hpi. The experimental schedule is shown in Fig. 6A.

The SARS-CoV-2 infectivity was measured by plaque assay. The results are shown in Fig. 6B. The plaque assay shows that in most cases a combination of effective single guide RNAs results in an increased ability to inhibit viral replication. All guide RNAs which were identified as efficient in the luciferase assay (Example 3) and tested in the combinational approach essay turned out to be highly efficient in suppressing the viral replication in living human epithelial lung cells. Thus, in principle all combinations of guide RNAs that showed a degradation efficiency close to 50% and below can be used for the combinational approach.

### Experimental design 2

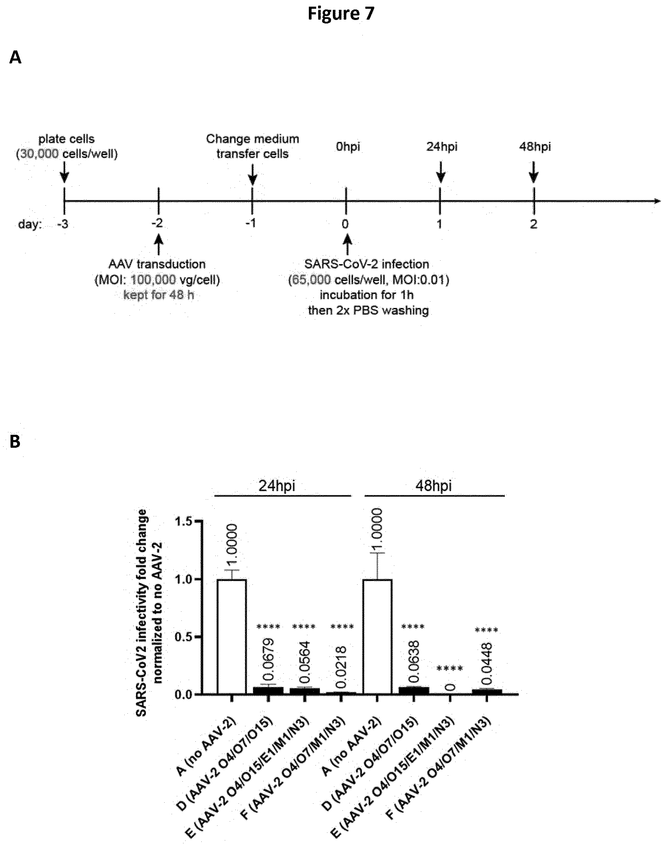

A new experimental design was developed (see Fig. 7A), involving a higher AAV titer (100,000 vg/cell per construct) to transduce Calu-3 cells (30,000 cells/well). After 48 hours, the AAV-transduced cells were further infected by SARS-CoV-2 virus, and the culture medium was collected at time points of 24 hpi and 48 hpi.

The SARS-CoV-2 infectivity was measured by plaque assay. The results are shown in Fig. 7B. All three samples (D-F) that were treated with different gRNA combinations showed significant effects as compared to untreated control sample. Combination D of 3 guide RNAs showed 93% reduction of SARS-CoV2 titer within 24 hours and 94% reduction within 48 hours. Combination F of 4 guide RNAs showed 98% reduction within 24 hours and 95% reduction within 48 hours. Combination E of 5 guide RNAs showed 94% reduction within 24 hours and 100% reduction within 48 hours.

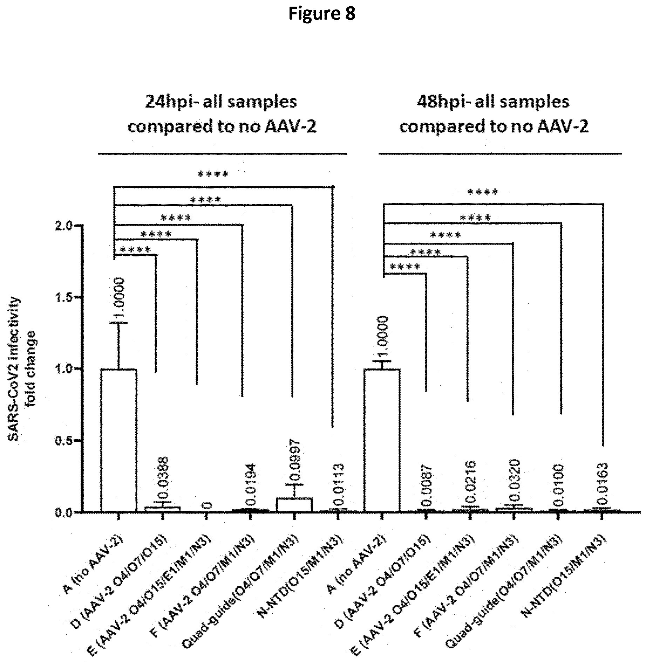

The experiment was repeated by also including multi-guide RNA constructs that could deliver several guide RNAs in one AAV. These included the quadguide construct of SEQ ID NO:42 and the construct of SEQ ID NO: 44 encoding Cas13d with NTD fused to its C-terminus. The results are shown in Fig. 8.

### REFERENCES

[1] Coronaviridae Study Group of the International Committee on Taxonomy of Viruses, "The species *Severe acute respiratory syndrome-related coronavirus*: classifying 2019-nCoV and naming it SARS-CoV-2", Nature Microbiology (5): 536-544, 2020.
[2] Wessels H-H et al., "Massively parallel Cas13 screens reveal principles for guide RNA design", Nature Biotechnology, 2020.
[3] Konermann S et al., "Transcriptome engineering with RNA-targeting Type VI-D CRISPR effectors", Cell (173(3)): 665-676, 2018
[4] Cong L, "Multiplex genome engineering using CRISPR/Cas systems", Science (339(6121)):819-23, 2013.
[5] Shmakov S et al., "Discovery and functional characterization of diverse class 2 CRISPR-Cas systems", Molecular Cell (69):385-397, 2015.
[6] Colella P et al., "Emerging issues in AAV-mediated in vivo gene therapy", Molecular Therapy: Methods & Clinical Development (8):87-104, 2018.
[7] Guggino WB et al., "AAV gene therapy for cystic fibrosis: current barriers and recent developments", Expert Opinion on Biological Therapy (17(10)):1265-1273, 2017.
[8] Moss RB et al., "Repeated Adeno-Associated Virus serotype 2 aerosol-mediated cystic fibrosis transmembrane regulator gene transfer to the lungs of patients with cystic fibrosis", CHEST (125(2)):509-521, 2004.
[9] Abbott TR et al., "Development of CRISPR as a prophylactic strategy to combat novel coronavirus and influenza", bioRxiv, 2020.
[10] Blanchard EL et al., "Treatment of influenza and SARS-CoV-2 infections via mRNA-encoded Cas13a in rodents", Nature Biotechnology, doi: 10.1038/s41587-021-00822-w, 2021.
[11] Grieger CJ & Samulski RJ, "Packaging capacity of adeno-associated virus serotypes: Impact of larger genomes on infectivity and postentry steps", Journal of Virology 79(15):9933-9944, 2005.
[12] Nguyen TM et al., "Virus against virus: a potential treatment for 2019-nCov (SARS-CoV-2) and other RNA viruses", Cell Research 30(3):189-190, 2020

## Claims

1. A guide RNA for use with a Cas13 protein, wherein the guide RNA target site is a sequence comprised by a SARS-CoV-2 genome, wherein the guide RNA sequence comprises a sequence selected from the group consisting of SEQ ID NOs: 4, 7, 11, 15, 23, 27, 31.

2. A nucleic acid molecule comprising a sequence encoding a Cas13d protein and a guide RNA expression cassette encoding a guide RNA according to claim 1 or more than one guide RNA according to claim 1 and comprising a U6 promotor.

3. The nucleic acid molecule according to claim 2, encoding guide RNAs comprising the sequences of SEQ ID NOs: 4, 7, and 15; SEQ ID NOs: 15, 23, and 31; SEQ ID NOs: 15, 27, and 31; SEQ ID NOs: 23, 27, and 31; SEQ ID NOs: 4, 15, 23, 27, and 31; SEQ ID NOs: 4, 7, 27, and 31; SEQ ID NOs: 4, 23, and 31; SEQ ID NOs: 7, 27, and 31; or SEQ ID NOs: 4, 7, 15, 23, 27, and 31, preferably SEQ ID NOs: 4, 7, 27, and 31.

4. The nucleic acid molecule according to any one of claims 2 or 3, wherein the nucleic acid molecule is a plasmid, preferably a single plasmid.

5. The nucleic acid molecule of any one of claims 2-4, wherein said Cas13d protein encoded by said sequence does not comprise a nuclear localization signal (NLS) and/ or is a fusion protein comprising an N-terminal binding domain (N-NTD) of the nucleocapsid protein of SARS-CoV-2.

6. The nucleic acid molecule according to any one of claims 4-5, wherein the nucleic acid molecule is obtainable by inserting a spacer sequence of a guide RNA according to any of claims 1-2 into plasmid pAAV2-U6-gRNA-CMV-Cas13d of SEQ ID NO:40; or by inserting at least one spacer sequence of at least one guide RNA according to any of claims 1-2 into plasmid pAAV2-U6-gRNA-CMV-Cas13d-array-triguide of SEQ ID NO:41, plasmid pAAV-U6-gRNA-quadguide-CMV-Cas13d-V3-basic of SEQ ID NO:42, plasmid pAAV-U6-gRNA-CMV-Cas13d-Sapl of SEQ ID NO:43, or plasmid pAAV-U6-gRNA-CMV-Cas13d-NTD-Aarl of SEQ ID NO:44.

7. An AAV vector comprising the nucleic acid molecule of any one of claims 2-6, preferably wherein the AAV vector is an AAV2 or AAV9 vector and/or wherein the AAV vector backbone has been reduced in size.

8. An adenoviral vector comprising the nucleic acid molecule of any one of claims 2-6.

9. A pharmaceutical composition comprising the AAV vector of claim 7 or the adenoviral vector of claim 8.

10. A pharmaceutical composition comprising at least one guide RNA of claim 1 and at least one mRNA encoding a Cas13 protein, preferably wherein said Cas13 protein encoded by said mRNA is a Cas13d and does not comprise a nuclear localization signal (NLS).

11. The pharmaceutical composition according to any one of claims 9-10, wherein said Cas13d protein encoded by said mRNA is a fusion protein comprising an N-terminal binding domain (N-NTD) of the nucleocapsid protein of SARS-CoV-2.

12. An AAV vector according to claim 7, an adenoviral vector according to claim 8, or a pharmaceutical composition according to any one of claims 9-11 for use in treating a human associated virus caused disease or syndrome in a patient in need thereof.

13. The AAV vector, the adenoviral vector, or the pharmaceutical composition for use according to claim 12, wherein the disease or syndrome is the result of an infection with a coronavirus that is genetically related to the group consisting of MERS-CoV, SARS-CoV and SARS-CoV-2, preferably wherein the disease is COVID-19.

14. The AAV vector, the adenoviral vector, or the pharmaceutical composition for use according to any one of claims 12-13, wherein the Cas13 upon expression cleaves the human-associated virus.

15. The AAV vector, the adenoviral vector, or the pharmaceutical composition for use according to any one of claims 12-14, wherein the AAV vector, the adenoviral vector, or the pharmaceutical composition is administered via the upper respiratory tract, preferably intranasally or intratracheally or in an aerosol composition through an inhaler or nebulizer, preferably through a ventilator.

16. The AAV vector, the adenoviral vector, or the pharmaceutical composition for use according to any one of claims 12-15, wherein the AAV vector, the adenoviral vector, or the pharmaceutical composition is administered to the myocardium.

## Patentansprüche

1. Eine Guide-RNA zur Verwendung mit einem Cas13 Protein, wobei die Guide-RNA-Zielstelle eine Sequenz ist, die von einem SARS-CoV-2-Genom umfasst ist, wobei die Guide-RNA-Sequenz eine Sequenz umfasst, die ausgewählt ist aus der Gruppe bestehend aus SEQ ID NOs: 4, 7, 11, 15, 23, 27, 31.

2. Ein Nukleinsäuremolekül umfassend eine Sequenz, die ein Cas13d Protein kodiert und eine Guide-RNA-Expressionskassette, die eine Guide-RNA nach Anspruch 1 oder mehr als eine Guide-RNA nach Anspruch 1 kodiert und einen U6 Promotor umfasst.

3. Das Nukleinsäuremolekül nach Anspruch 2, das Guide-RNAs kodiert, die die Sequenzen der SEQ ID NOs: 4, 7 und 15, SEQ ID NOs: 15, 23 und 31, SEQ ID NOs: 15, 27 und 31, SEQ ID NOs: 23, 27 und 31, SEQ ID NOs: 4, 15, 23, 27 und 31, SEQ ID NOs: 4, 7, 27 und 31, SEQ ID NOs: 4, 23 und 31, SEQ ID NOs: 7, 27 und 31, SEQ ID NOs: 4, 7, 15, 23, 27 und 31, bevorzugt SEQ ID NOs: 4, 7, 27 und 31 umfassen.

4. Das Nukleinsäuremolekül nach einem der Ansprüche 2 oder 3, wobei das Nukleinsäuremolekül ein Plasmid ist, bevorzugt ein einzelnes Plasmid.

5. Das Nukleinsäuremolekül nach einem der Ansprüche 2-4, wobei jenes Cas13d Protein, das von jener Sequenz kodiert wird, kein Nukleares Lokalisierungssignal (NLS) umfasst und/oder ein Fusionsprotein ist, das eine N-terminale Bindedomäne (N-NTD) des Nukleocapsidproteins von SARS-CoV-2 umfasst.

6. Das Nukleinsäuremolekül nach einem der Ansprüche 4-5, wobei das Nukleinsäuremolekül durch Insertion einer Spacer-Sequenz einer Guide-RNA nach einem der Ansprüche 1-2 in das Plasmid pAAV2-U6-gRNA-CMV-Cas13d der SEQ ID NO: 40 erhalten werden kann, oder durch Insertion mindestens einer Spacer-Sequenz mindestens einer Guide-RNA nach einem der Ansprüche 1-2 in das Plasmid pAAV2-U6-gRNA-CMV-Cas13d-array-triguide der SEQ ID NO: 41, Plasmid pAAV-U6-gRNA-quadguide-CMV-Cas13d-V3-basic der SEQ ID NO: 42, Plasmid pAAV-U6-gRNA-CMV-Cas13d-Sapl der SEQ ID NO: 43 oder Plasmid pAAV-U6-gRNA-CMV-Cas13d-NTD-Aarl der SEQ ID NO: 44.

7. Ein AAV-Vektor umfassend das Nukleinsäuremolekül nach einem der Ansprüche 2-6, bevorzugt wobei der AAV-Vektor ein AAV2- oder AAV8-Vektor ist und/oder wobei das AAV-Vektor-Rückgrat in der Größe reduziert wurde.

8. Ein Adenovirus-Vektor umfassend das Nukleinsäuremolekül nach einem der Ansprüche 2-6.

9. Eine pharmazeutische Zusammensetzung umfassend den AAV-Vektor nach Anspruch 7 oder den Adenovirus-Vektor nach Anspruch 8.

10. Eine pharmazeutische Zusammensetzung umfassend mindestens eine Guide-RNA von Anspruch 1 und mindestens eine mRNA, die ein Cas13 Protein kodiert, bevorzugt wobei jenes Cas13 Protein, das von jener mRNA kodiert wird, ein Cas13d ist und kein Nukleares Lokalisationssignal (NLS) umfasst.

11. Die pharmazeutische Zusammensetzung nach einem der Ansprüche 9-10, wobei jenes Cas13d Protein, das von jener mRNA kodiert wird, ein Fusionsprotein ist, das eine N-terminale Bindedomäne (N-NTD) des Nukleocapsidproteins von SARS-CoV-2 umfasst.

12. Ein AAV-Vektor nach Anspruch 7, ein Adenovirus-Vektor nach Anspruch 8 oder eine pharmazeutische Zusammensetzung nach einem der Ansprüche 9-11 zur Verwendung bei der Behandlung einer mit dem Menschen assoziierten und von einem Virus ausgelösten Erkrankung oder eines solchen Syndroms in einem Patienten, der daran Bedarf hat.

13. Der AAV-Vektor, der Adenovirus-Vektor oder die pharmazeutische Zusammensetzung zur Verwendung nach Anspruch 12, wobei die Erkrankung oder das Syndrom das Resultat einer Infektion mit einem Coronavirus ist, der genetisch mit der Gruppe verbunden ist, die besteht aus MERS-CoV, SARS-CoV und SARS-CoV-2, bevorzugt wobei die Erkrankung COVID-19 ist.

14. Der AAV-Vektor, der Adenovirus-Vektor oder die pharmazeutische Zusammensetzung zur Verwendung nach einem der Ansprüche 12-13, wobei das Cas13 nach Expression das mit dem Menschen assoziierte Virus schneidet.

15. Der AAV-Vektor, der Adenovirus-Vektor oder die pharmazeutische Zusammensetzung zur Verwendung nach einem der Ansprüche 12-14, wobei der AAV-Vektor, der Adenovirus-Vektor oder die pharmazeutische Zusammensetzung über den oberen respiratorischen Trakt verabreicht wird, bevorzugt intranasal oder intratracheal oder in einer Aerosol-Zusammensetzung durch einen Inhalator oder Zerstäuber, bevorzugt durch ein Beatmungsgerät.

16. Der AAV-Vektor, der Adenovirus-Vektor oder die pharmazeutische Zusammensetzung zur Verwendung nach einem der Ansprüche 12-15, wobei der AAV-Vektor, der Adenovirus-Vektor oder die pharmazeutische Zusammensetzung an das Myokard verabreicht wird.

## Revendications

1. ARN guide pour utilisation avec une protéine Cas13, dans lequel le site cible de l'ARN guide est une séquence composée par un génome de SARS-CoV-2, dans lequel la séquence d'ARN guide comprend une séquence choisie dans le groupe consistant en SEQ ID N° : 4, 7, 11, 15, 23, 27, 31.

2. Molécule d'acide nucléique comprenant une séquence codant une protéine Cas13d et une cassette d'expression d'ARN guide codant un ARN guide selon la revendication 1 ou plus d'un ARN guide selon la revendication 1 et comprenant un promoteur U6.

3. Molécule d'acide nucléique selon la revendication 2, codant des ARN guides comprenant les séquences de SEQ ID N° : 4, 7 et 15 ; SEQ ID N° : 15, 23 et 31 ; SEQ ID N° : 15, 27 et 31 ; SEQ ID N° : 23, 27 et 31 ; SEQ ID N° : 4, 15, 23, 27 et 31 ; SEQ ID N° : 4, 7, 27 et 31 ; SEQ ID N° : 4, 23 et 31 ; SEQ ID N° : 7, 27 et 31 ; ou SEQ ID N° : 4, 7, 15, 23, 27 et 31, de préférence SEQ ID N° : 4, 7, 27 et 31.

4. Molécule d'acide nucléique selon l'une quelconque des revendications 2 ou 3, la molécule d'acide nucléique étant un plasmide, de préférence un seul plasmide.

5. Molécule d'acide nucléique selon l'une quelconque des revendications 2 à 4, dans laquelle ladite protéine Cas13d codée par ladite séquence ne comprend pas de signal de localisation nucléaire (NLS) et/ou est une protéine de fusion comprenant un domaine de liaison N-terminal (N-NTD) de la protéine de nucléocapside de SARS-CoV-2.

6. Molécule d'acide nucléique selon l'une quelconque des revendications 4 et 5, la molécule d'acide nucléique pouvant être obtenue en insérant une séquence d'espacement d'un ARN guide selon l'une quelconque des revendications 1 et 2 dans le plasmide pAAV2-U6-gRNA-CMV-Cas13d de SEQ ID N° : 40 ; ou en insérant au moins une séquence d'espacement d'au moins un ARN guide selon l'une quelconque des revendications 1 et 2 dans le plasmide pAAV2-U6-gRNA-CMV-Cas13d-réseau-triguide de SEQ ID N° : 41, le plasmide pAAV-U6-gRNA-quadguide-CMV-Cas13d-V3-basique de SEQ ID N° : 42, le plasmide pAAV-U6-gRNA-CMV-Cas13d-Sapl de SEQ ID N° : 43, ou le plasmide pAAV-U6-gRNA-CMV-Cas13d-NTD-Aarl de SEQ ID N° : 44.

7. Vecteur AAV comprenant la molécule d'acide nucléique de l'une quelconque des revendications 2 à 6, de préférence le vecteur AAV étant un vecteur AAV2 ou AAV9 et/ou dans lequel la taille du squelette du vecteur AAV a été réduite.

8. Vecteur adénoviral comprenant la molécule d'acide nucléique de l'une quelconque des revendications 2 à 6.

9. Composition pharmaceutique comprenant le vecteur AAV de la revendication 7 ou le vecteur adénoviral de la revendication 8.

10. Composition pharmaceutique comprenant au moins un ARN guide de la revendication 1 et au moins un ARNm codant une protéine Cas13, de préférence dans laquelle ladite protéine Cas13 codée par ledit ARNm est une Cas13d et ne comprend pas de signal de localisation nucléaire (NLS).

11. Composition pharmaceutique selon l'une quelconque des revendications 9 et 10, dans laquelle ladite protéine Cas13d codée par ledit ARNm est une protéine de fusion comprenant un domaine de liaison N-terminal (N-NTD) de la protéine de nucléocapside de SARS-CoV-2.

12. Vecteur AAV selon la revendication 7, vecteur adénoviral selon la revendication 8, ou composition pharmaceutique selon l'une quelconque des revendications 9 à 11, pour utilisation dans le traitement d'une maladie ou d'un syndrome causée par un virus associé humain chez un patient qui en a besoin.

13. Vecteur AAV, vecteur adénoviral ou composition pharmaceutique pour utilisation selon la revendication 12, dans lequel ou laquelle la maladie ou le syndrome est le résultat d'une infection par un coronavirus qui est génétiquement lié au groupe consistant en MERS-CoV, SARS-CoV et SARS-CoV-2, de préférence dans lequel ou laquelle la maladie est la COVID-19.

14. Vecteur AAV, vecteur adénoviral ou composition pharmaceutique pour utilisation selon l'une quelconque des revendications 12 et 13, dans lequel ou laquelle la Cas13, lors de l'expression, clive le virus associé humain.

15. Vecteur AAV, vecteur adénoviral, ou composition pharmaceutique pour utilisation selon l'une quelconque des revendications 12 à 14, le vecteur AAV, le vecteur adénoviral, ou la composition pharmaceutique étant administrée via les voies respiratoires supérieures, de préférence par voie intranasale ou intratrachéale, ou dans une composition d'aérosol à travers un inhalateur ou un nébuliseur, de préférence à travers un ventilateur.

16. Vecteur AAV, vecteur adénoviral ou composition pharmaceutique pour utilisation selon l'une quelconque des revendications 12 à 15, le vecteur AAV, le vecteur adénoviral ou la composition pharmaceutique étant administrée au myocarde.
